(19) Europäisches Patentamt
European Patent Office
Office européen des brevets

(11) **EP 1 826 193 A1**

(12) **EUROPÄISCHE PATENTANMELDUNG**

| | |
|---|---|
| (43) Veröffentlichungstag:<br>**29.08.2007 Patentblatt 2007/35** | (51) Int Cl.:<br>***C07C 29/74*** *(2006.01)*     ***C07C 31/08*** *(2006.01)*<br>***C07C 43/04*** *(2006.01)*     ***C07C 41/06*** *(2006.01)* |

(21) Anmeldenummer: **06124922.3**

(22) Anmeldetag: **28.11.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **25.01.2006 DE 102006003492**

(71) Anmelder: **Oxeno Olefinchemie GmbH**
**45772 Marl (DE)**

(72) Erfinder:
• **Rix, Armin**
**45770, Marl (DE)**

• **Höper, Frank**
**45721, Haltern am See (DE)**
• **Praefke, Jochen**
**45739, Oer-Erkenschwick (DE)**
• **Büschken, Wilfried**
**45721, Haltern am See (DE)**

(74) Vertreter: **Hirsch, Hans-Ludwig et al**
**Degussa GmbH**
**Intellectual Property Management**
**PATENTE & MARKEN**
**Bau 1042 / PB 15**
**45764 Marl (DE)**

(54) **Verfahren zur Entwässerung von Ethanol**

(57) Die Erfindung betrifft ein Verfahren zur Herstellung eines wasserarmen Ethanols aus mindestens zwei Strömen von wasserreicherem Ethanol, die einen unterschiedlichen Wassergehalt aufweisen, durch Entwässerung an Membranen sowie die Verwendung des wasserarmen Ethanols zur Herstellung von Ethyl-tert.-butylether (ETBE).

**EP 1 826 193 A1**

**Beschreibung**

Die vorliegende Erfindung betrifft ein Verfahren zur Entwässerung von Ethanol, insbesondere zur Entwässerung von Ethanol, welches zur Herstellung von Ethyl-tert.-butylether (ETBE) durch Umsetzung von Ethanol mit einem Isobuten-haltigen Kohlenwasserstoffgemisch eingesetzt wird. Das zu entwässernde Ethanol kann dabei teilweise von einem bei einem ETBE-Prozess anfallenden ethanolhaltigen $C_4$-Kohlenwasserstoffstrom stammen.

ETBE ist eine Komponente zur Erhöhung der Oktanzahl in Kraftstoffen für Ottomotoren. ETBE wird zunehmend anstelle von MTBE als Oktanzahlverbesserer eingesetzt, da über die Verwendung von MTBE in Kraftstoffen wegen möglicher Grundwasser-Kontamination diskutiert wird. Zudem wird der Einsatz von ETBE in einigen Ländern, wie z. B. Deutschland gefördert, wenn zur Herstellung von ETBE Bioalkohol, also Ethanol, hergestellt aus nachwachsenden Rohstoffen (Pflanzen), eingesetzt wird.

ETBE kann aus $C_4$-Olefingemischen, beispielsweise aus $C_4$-Schnitten aus Steamcrackern, gewonnen werden. Diese Gemische bestehen im Wesentlichen aus Butadien, den Monoolefinen, Isobuten, 1-Buten und den beiden 2-Butenen sowie den gesättigten Kohlenwasserstoffen Isobutan und n-Butan. Übliche weltweit ausgeübte Aufarbeitungs-verfahren für solche $C_4$-Schnitte umfassen folgende Schritte: Zuerst wird der größte Teil des Butadiens entfernt. Kann Butadien gut vermarktet werden oder besteht ein Eigenverbrauch, wird es z. B. durch Extraktion oder Extraktivdestillation abgetrennt. Im anderen Fall wird das Butadien möglichst vollständig, zumindest aber bis zu Konzentrationen von 1 bis 0,1 Massen-% selektiv zu linearen Butenen hydriert. Zurück bleibt in beiden Fällen ein Kohlenwasserstoffgemisch (häufig als Raffinat I bzw. hydriertes Crack-$C_4$ bezeichnet), das neben den gesättigten Kohlenwasserstoffen (n-Butan und Isobutan) die Olefine (Isobuten, 1-Buten und 2-Butene) enthält. Das darin enthaltene Isobuten wird mit Ethanol zu ETBE umgesetzt. Die destillative Aufarbeitung ergibt ETBE und ein Ethanolhaltiges $C_4$-Kohlenwasserstoffgemisch, aus dem durch Extraktion mit Wasser Ethanol entfernt wird.

In der Technik erfolgt die Umsetzung von Isobuten bzw. Isobuten-haltigen Kohlenwasserstoffströmen meistens an festen sauren Katalysatoren, wie beispielsweise an sauren Ionenaustauscherharzen.

Bei dieser Umsetzung wird durch Wasser die Aktivität des Katalysators sehr verringert und somit die Raum-Zeit-Ausbeute für ETBE herabgesetzt. Darüber hinaus entsteht aus Isobuten in Gegenwart von Wasser als Nebenprodukt tert.-Butanol (TBA). In der Technik werden daher bei der Herstellung von ETBE wasserarme Ethanole eingesetzt, insbesondere solche, die einen Wassergehalt von unter 1 Massen-% aufweisen.

Die handelsüblichen Ethanolqualitäten weisen einen Wassergehalt zwischen 5 und weniger als 1 Massen-% auf. Das Homoazeotrop aus Ethanol und Wasser hat noch einen Wassergehalt von 4,4 Massen-%. Trockenere Ethanole, insbesondere solche mit einem Wassergehalt von unter 1 Massen-%, können nur mit höherem Aufwand hergestellt werden.

Ein technisches Verfahren zur Entwässerung von Ethanol ist die Azeotropdestillation mit einem Schleppmittel, das mit Ethanol und Wasser ein ternäres Heterominimumazeotrop bildet. Als Schleppmittel werden beispielsweise Heptan, Cyclohexan oder Benzol eingesetzt. Ein allgemeines Fließschema einer solchen Anlage und Literaturhinweise findet man in Ullmann's Encyclopedia of Technical Chemistry, Vol. 9, pages 634- 635, 5th Edition. Der Hauptnachteil dieser Entwässerungsverfahren liegt in den hohen Betriebskosten.

Ein weiteres Verfahren zur Trocknung von Ethanol ist die Wasserabtrennung durch Pervaporation an Membranen (z. B. U. Sander, H. Janssen, Industrial application of vapour permeation, Journal of Membrane Science, 61 (1991), S. 113 bis 129, Elsevier Science Publishers B. V, Amsterdam; A. H. Ballweg, H. E. A. Brüschke, W. H. Schneider, G. F. Tusel, Pervaporation Membranes, Proceedings of Fifth International Alcohol Fuel Technology 1982, S. 97 bis 106; H. E. A. Brüschke, State of Art of Pervaporation, Proceeding of Third International Conference on Pervaporation 1988, S. 2 bis 11). Dabei wird das abzutrennende Wasser zusammen mit Ethanol dampfförmig als Permeat erhalten. Um eine möglichst weitgehende Wasserabtrennung zu erreichen, wird die Pervaporation in mehreren hintereinandergeschalteten Membranmodulen durchgeführt, wobei jeweils das Retentat eines Moduls das Einsatzgemisch des nächsten Moduls ist. Dieses Verfahren ist besonders für die Entwässerung von Ethanolen mit einem geringen Wassergehalt (1 bis 10 Massen-%) geeignet. Die anfallenden Permeate können in einer Kolonne zum Ethanol/Wasser-Azeotrop aufgearbeitet werden, das in eine Membraneinheit zurückgeführt werden kann. Der Hauptnachteil dieses Verfahrens liegt im hohen Kapitaleinsatz für die Membranmodule.

In den meisten ETBE-Anlagen gibt es üblicherweise mindestens zwei verschiedene Ethanolströme, nämlich das Einsatzethanol (Ethanol aus einer anderen Anlage oder Zukaufware) und mindestens einen aus einem ethanolhaltigen $C_4$-Gemisch durch Extraktion und Destillation zurückgewonnenen Ethanolstrom. Das Frischethanol weist in der Regel einen Wassergehalt von größer 0 bis kleiner 1 bis ca. 5 Massen-%, das zurückgewonnene meistens einen Wassergehalt von 5 bis 12 Massen-% auf. Üblicherweise werden diese Ströme vor der Entwässerung zusammengeführt.

Aufgabe der vorliegenden Erfindung war es, ein alternatives Verfahren für die Entwässerung von zwei Ethanol-Strömen mit unterschiedlichem Wassergehalt bereitzustellen, welches vorzugsweise die Nachteile der bekannten Entwässerungsverfahren, bei denen die beiden Ethanolströme vor der Entwässerung gemischt werden, vermeidet.

Überraschenderweise wurde nun gefunden, dass die Membranfläche, die für die Abtrennung des Wassers aus beiden Ethanolströmen notwendig ist, verringert werden kann, wenn die beiden Ströme mit unterschiedlichem Wassergehalt in separaten, für die individuelle Zulaufkonzentration optimierten Membraneinheiten aufgearbeitet werden. Die Nebenaggregate (Kondensation, Kältekreislauf, Vakuum-Erzeugung) können gegebenenfalls von den beiden Membraneinheiten gemeinsam genutzt werden.

Gegenstand der vorliegenden Erfindung ist deshalb ein Verfahren zur Herstellung eines wasserärmeren Ethanols aus mindestens zwei Strömen von wasserreicherem Ethanol, die einen unterschiedlichen Wassergehalt aufweisen, durch Entwässerung an Membranen, welches dadurch gekennzeichnet ist, dass die Ethanolströme mit unterschiedlichen Wassergehalten getrennt in verschiedene Membraneinheiten eingeleitet werden, wobei die Ethanolströme in den Membraneinheiten jeweils in wasserärmeres Ethanol als Retentat und wasserreicheres Permeat getrennt werden und der Retentatstrom aus zumindest einer Membraneinheit zur Trocknung eines Ethanolstroms mit einem höheren Wassergehalt mit dem Zustrom und/oder dem Retentatstrom zumindest einer anderen Membraneinheit zur Trocknung eines Ethanolstroms vereinigt wird, wobei nur Ströme vereinigt werden, die einen Unterschied in der Ethanolkonzentration von maximal 1 Massen-% absolut aufweisen.

Ebenfalls Gegenstand der vorliegenden Erfindung ist eine Zusammensetzung von Ethanol und Wasser mit einem Wassergehalt von kleiner 1 Massen-%, welches nach dem erfindungsgemäßen Verfahren hergestellt wurde.

Außerdem ist Gegenstand der vorliegenden Erfindung die Verwendung von Zusammensetzungen enthaltend Ethanol, hergestellt gemäß dem erfindungsgemäßen Verfahren, als Edukt zur Herstellung von ETBE.

Weiterhin kann die Erfindung zur Entwässerung nur eines Ethanolstroms genutzt werden, wobei das durch destillative Aufarbeitung der bei der Trennung anfallenden Permeate gewonnene Ethanol/Wasser-Gemisch formal den zweiten Ethanolstrom darstellt.

Die vorliegende Erfindung weist den Vorteil auf, dass durch die Verringerung der für die Wasserabtrennung notwendigen Membranflächen der Kapitaleinsatz und durch Verringerung der regelmäßig zu ersetzenden Membranfläche auch die Betriebskosten gesenkt werden können.

Nachfolgend wird das erfindungsgemäße Verfahren beispielhaft beschrieben, ohne dass die Erfindung, deren Schutzbereich sich aus den Ansprüchen und der gesamten Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche und Einzelwerte zum Offenbarungsgehalt der vorliegenden Erfindung gehören, ohne dass diese aus Gründen der besseren Übersichtlichkeit explizit genannt worden sind.

Das erfindungsgemäße Verfahren zur Herstellung von wasserärmerem Ethanol aus mindestens zwei Strömen von wasserreicherem Ethanol, die einen unterschiedlichen Wassergehalt aufweisen, durch Entwässerung an Membranen, zeichnet sich dadurch aus, dass die Ethanolströme mit unterschiedlichen Wassergehalten getrennt in verschiedene Membraneinheiten eingeleitet werden, wobei die Ethanolströme in den Membraneinheiten jeweils in wasserärmeres Ethanol als Retentat und wasserreicheres Permeat getrennt werden und der Retentatstrom aus zumindest einer Membraneinheit zur Trennung eines Ethanolstroms mit einem höheren Wassergehalt mit dem Zustrom und/oder dem Retentatstrom zumindest einer anderen Membraneinheit zur Trennung eines Ethanolstroms vereinigt wird, wobei nur Ströme vereinigt werden, die einen Unterschied in der Ethanolkonzentration von maximal 1 Massen-% absolut aufweisen.

Vorzugsweise werden nur solche Retentatströme mit Zuströmen und/oder Retentatströmen anderer Membraneinheiten vereinigt, die einen Unterschied in der absoluten Ethanolkonzentration von maximal 0,5 Massen-%, bevorzugt von maximal 0,2 Massen-% und ganz besonderes bevorzugt von 0 bis 0,2 Massen-%, insbesondere von 0,001 bis 0,1 Massen-% aufweisen. Erst wenn der Unterschied der Wasserkonzentration der beiden zu vereinigenden Ströme kleiner als 1 Massen-% wird, können die Vorteile der getrennten Aufarbeitung die höheren Investitionskosten nicht mehr ausreichend ausgleichen, so dass dann eine gemeinsame Aufarbeitung bevorzugt ist.

Es kann vorteilhaft sein, wenn der Retentatstrom aus zumindest einer Membraneinheit zur Trennung eines Ethanolstroms mit einem höheren Wassergehalt mit dem Zustrom und/oder dem Retentatstrom zumindest einer Membraneinheit zur Trennung eines Ethanolstroms mit einem niedrigeren Wassergehalt vereinigt wird. Vorzugsweise sind zwei wasserreiche Ethanolströme mit unterschiedlichem Wassergehalt vorhanden, so dass das Retentat aus der Membraneinheit, in der der Ethanolstroms mit dem höheren Wassergehalt entwässert wird, mit dem Zustrom zur und/oder dem Retentatstrom aus der Membraneinheit vereinigt werden kann, in der der Ethanolstrom mit dem niedrigeren Wassergehalt entwässert wird. Unter wasserärmerem Ethanol wird im Rahmen der vorliegenden Erfindung insbesondere Ethanol oder eine Ethanol aufweisende Zusammensetzung verstanden welches/welche einen Wassergehalt aufweist, der kleiner ist als der Wassergehalt der wasserreicheren Ströme von Ethanol und der besonders bevorzugt einen Wassergehalt aufweist, der kleiner ist als der Wassergehalt des Ethanol/Wasser-Azeotrops bei Normaldruck.

Im Rahmen der vorliegenden Erfindung bedeutet der Begriff Membraneinheit zumindest eine Membran bzw. eine ein Membranmodul aufweisende Einheit. Eine Membraneinheit kann z. B. ein Membranmodul oder mehrere hintereinander- oder parallelgeschaltete Membranmodule oder eine Anordnung von parallel und hintereinandergeschalteten Membranmodulen aufweisen. Als Membranmodule können handelsübliche Module eingesetzt werden. In den Membranmodulen können eine oder mehrere Membrane vorhanden sein.

Die in dem erfindungsgemäßen Verfahren eingesetzten zumindest zwei wasserreicheren Ethanolströme mit unterschiedlichen Wassergehalten unterscheiden sich in ihrer Wasserkonzentration vorzugsweise um mehr als 1 Massen-% absolut, bevorzugt um 1,1 bis 10 Massen-% absolut und besonders bevorzugt um 2 bis 5 Massen-% absolut. Bei den wasserreicheren Ethanolströmen kann es sich z. B. um Ethanol aus einer Ethanolherstellung, beispielsweise aus der Bioethanolherstellung oder einer industriellen Ethanolherstellung, handeln. Der unterschiedliche Wassergehalt kann bei diesen Frischalkoholen beispielsweise aus einem unterschiedlichen Herstellungsverfahren resultieren. Bei den wasserreichen Strömen kann es sich aber auch um Rückführströme handeln, wie sie in Verfahren, bei denen Ethanol als Edukt eingesetzt wird, anfallen können. Bei den wasserreicheren Ethanolströmen kann es sich auch um einen oder mehrere Frischalkoholströme und einen oder mehrere Rückführströme handeln. Da das erfindungsgemäße Verfahren insbesondere zur Aufbereitung von in ETBE-Synthesen bzw. - Verfahren einzusetzenden Ethanolströmen geeignet ist, werden in dem erfindungsgemäßen Verfahren bevorzugt als wasserreichere Ethanolströme Frischalkohol und zumindest ein wasserreicher Ethanolstrom, der aus einer ETBE-Anlage bzw. einem ETBE-Verfahren stammt, eingesetzt.

In dem erfindungsgemäßen Verfahren kann die Abtrennung von Wasser aus den wasserreicheren Ethanolströmen durch Umkehr-Osmose (flüssiger Zulauf und flüssiges Retentat; flüssiges Permeat), Dampfpermeation (dampfförmiger Eingangsstrom und Retentat; dampfförmiges Permeat) oder durch Pervaporation (flüssiger Eingangsstrom und flüssiges Retentat; dampfförmiges Permeat) erfolgen. Weiterhin ist auch eine Abtrennung durch gleichzeitige Pervaporation und Dampfpermeation möglich. Besonders bevorzugt erfolgt die Abtrennung durch Pervaporation. In dem erfindungsgemäßen Verfahren ist es möglich, dass zwei oder mehrere der vorhandenen Membraneinheiten nach unterschiedlichem Trennprinzip oder gleichem Trennprinzip arbeiten. Vorzugsweise wird in allen Membraneinheiten das gleiche Trennprinzip, also insbesondere die Pervaporation, eingesetzt.

In dem erfindungsgemäßen Verfahren können handelsübliche, vorzugsweise hydrophile Membrane eingesetzt werden. Die Membrane können z. B. Polymermembrane oder anorganische Membrane oder auch Verbundmembrane, die sowohl anorganische als auch organische Werkstoffe aufweisen, sein. Im erfindungsgemäßen Verfahren können beispielsweise Polymermembrane der Firmen Sulzer Chemtech, CM-Celfa, GKSS oder Sophisticated Systems (Polyimidmembran), wie beispielsweise Typ Pervap 2200, Pervap 2201, Pervap 2256, Pervap 2510 von Sulzer oder Typ 2SDP-H018 von Sophisticated Systems, eingesetzt werden. Als anorganische Membrane können beispielsweise verwendet werden: SMS (Sulzer Chemtech), Silica (Pervatech), NaA (Mitsui oder Smart Chemical).

Die erfindungsgemäße Entwässerung erfolgt an den anorganischen Membranen vorzugsweise bei einer Temperatur im Bereich von 20 bis 200 °C und an den Polymermembranen vorzugsweise im Temperaturbereich von 20 bis 150 °C. Bevorzugt wird die Entwässerung an den Membranen bei einer Temperatur von 20 bis 150 °C, besonders bevorzugt bei einer Temperatur von 40 bis 140 °C und ganz besonders bevorzugt bei einer Temperatur von 60 bis 130 °C durchgeführt. Die Abtrennung an den Membranen kann an jeder Membran, in jedem Modul oder in jeder Membraneinheit bei unterschiedlicher oder gleicher Temperatur erfolgen. Insbesondere wenn wasserreichere Ethanolströme mit dem erfindungsgemäßen Verfahren aufgearbeitet werden sollen, von denen einer ein Frischalkoholstrom und der andere ein Rückführstrom ist, kann es vorteilhaft sein, den Rückführstrom mit einer anderen Temperatur als den Frischalkohol auf die Membran zu fahren, um z. B. Wärmeenergie, die dem Rückführstrom inne wohnt, nicht zu verlieren.

Bei der Entwässerung beträgt der Druck auf der Retentatseite der Membran vorzugsweise von 0,05 bis 3 MPa, bevorzugt von 0,1 bis 2 MPa. Der Druck auf der Permeatseite der Membran beträgt vorzugsweise von 0,0001 bis 0,1 MPa, bevorzugt 0,001 bis 0,01 MPa.

Bei Polymermembranen beträgt der Differenzdruck vorzugsweise von 0,001 bis 2 MPa und bei anorganischen Membranen vorzugsweise von 0,001 bis 3 MPa. Bevorzugt wird die erfindungsgemäße Entwässerung an den Membranen bei einem Differenzdruck von 0,001 bis 2 MPa und besonders bevorzugt von 0,1 bis 0,8 MPa durchgeführt. Der Differenzdruck kann an allen eingesetzten Membranen, Membranmodulen oder Membraneinheiten gleich oder unterschiedlich sein.

Der Druck bzw. der Differenzdruck kann auf unterschiedliche Weise erzeugt werden. Der Differenzdruck kann zum Beispiel dadurch erzeugt werden, dass auf der Zustromseite ein Überdruck erzeugt wird. Der Differenzdruck kann aber z. B. auch dadurch erzeugt werden, dass auf der Permeatseite der Membran ein Unterdruck, insbesondere zusätzlich ein Unterdruck (Vakuum) erzeugt wird. Vorzugsweise wird der Differenzdruck dadurch erzeugt, dass auf der Permeatseite zusätzlich ein Unterdruck erzeugt wird, da auf diese Weise ein Kompaktieren der Membrane vermindert werden kann. Zur Erzeugung eines Unterdrucks kann in dem erfindungsgemäßen Verfahren jede Membran oder Membraneinheit mit einem eigenen Unterdrucksystem ausgerüstet sein. Um den Kapitaleinsatz klein zu halten und Betriebskosten zu sparen, ist es aber bevorzugt, alle eingesetzten Membrane oder Membraneinheiten permeatseitig an ein gemeinsames Unterdrucksystem anzuschließen.

Der spezifische Massenstrom durch die Membran (kg Permeat je Quadratmeter Membranoberfläche je Stunde) ist abhängig von der Wasserkonzentration des Retentats. Als auf die Konzentration bezogener Massenstrom wird der extrapolierte Reinwasserfluss herangezogen. Er beträgt vorzugsweise von 1 bis 100 $kg/(m^2 \cdot h)$, bevorzugt von 5 bis 30 $kg/(m^2.h)$. Der bevorzugte Reinwasserfluss kann durch Auswahl einer geeigneten Membran und Auswahl eines entsprechenden Differenzdrucks eingestellt werden. Die Auswahl kann durch einfache Vorversuche erfolgen.

Es versteht sich von selbst, dass alle Membranmodule bei gleichen oder unterschiedlichen Betriebsparametern betrieben werden können, unter der Voraussetzung, dass diese in den oben genannten Bereichen liegen.

Wird im erfindungsgemäßen Verfahren an den Membranen das Trennprinzip der Dampfpermeation oder Pervaporation eingesetzt, wird ein dampfförmiges Permeat gewonnen. Je nach weiterem Verwendungszweck kann es vorteilhaft sein, das Permeat durch Kondensation in einem Wärmeübertrager zu kondensieren.

Die in dem erfindungsgemäßen Verfahren bei der Entwässerung in den Membraneinheiten anfallenden wasserreichen Permeate können getrennt oder zusammen aufgearbeitet werden. Vorzugsweise erfolgt die Aufarbeitung der anfallenden wasserreichen Permeate gemeinsam. Die Aufarbeitung kann beispielsweise durch Trennung an Membranen, durch Destillation oder durch Destillation unter Verwendung eines Schleppmittel erfolgen. Eine gemeinsame Aufarbeitung der Permeate ist insbesondere dann bevorzugt, wenn die Permeate einen Wassergehalt aufweisen, der größer ist als die Konzentration des Ethanol/Wasser-Azeotrops, da in einem solchen Fall eine einfache Aufkonzentration des Permeats bzw. der vereinigten Permeatströme mittels Destillation möglich ist. In einer bevorzugten Ausführungsform werden die Permeate gemeinsam oder getrennt destillativ in Wasser und ein Destillat, bestehend aus dem Ethanol/Wasser-Azeotrop oder bestehend aus dem Ethanol/Wasser-Azeotrop und zusätzlichem Wasser getrennt und das Destillat wird als wasserreicher Ethanolstrom in eine der Membraneinheiten des Verfahrens zurückgeführt.

Ist das erfindungsgemäße Verfahren ein Teilverfahren in einem Prozess zur Herstellung von ETBE, so kann es insbesondere vorteilhaft sein, wenn die Permeate gemeinsam oder getrennt zusammen mit einem Ethanol-haltigen wässrigen Extrakt aus einer ETBE-Anlage destillativ aufgearbeitet werden und anschließend das an Ethanol reiche Destillat als wasserreicher Ethanolstrom in eine der Membraneinheiten des erfindungsgemäßen Verfahrens zurückgeführt wird.

Die aus den Membraneinheiten erhaltenen Retentate können entweder getrennt oder gemeinsam verwendet werden oder getrennt oder gemeinsam einer Aufarbeitung zugeführt werden. Eine solche Aufarbeitung kann z. B. aus einer weiteren Entwässerung in einer Membraneinheit oder aus einer destillativen Aufarbeitung bestehen. Es kann vorteilhaft sein die Retentate aus den vorhandenen Membraneinheiten zu vereinigen, wenn sich deren Wasserkonzentrationen um weniger als 1 Massen-%, vorzugsweise um weniger als 0,5 Massen-%, besonders bevorzugt um weniger als 0,2 Massen-% und ganz besonderes bevorzugt von 0 bis 0,2 Massen-%, insbesondere von 0,001 bis 0,1 Massen-% unterscheiden. Erst wenn der Unterschied der Wasserkonzentration der beiden zu vereinigenden Ströme kleiner als 1 Massen-% wird, können die Vorteile der getrennten Aufarbeitung die höheren Investitionskosten nicht mehr ausreichend ausgleichen, so dass dann eine gemeinsame Aufarbeitung bevorzugt ist.

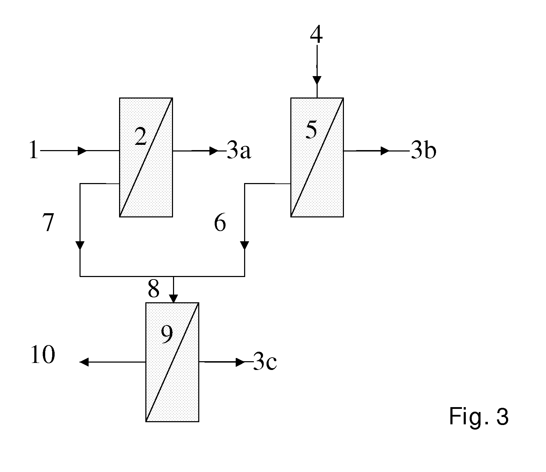

Wenn die gewünschte Spezifikation des wasserärmeren Ethanols in den Retentaten noch nicht erreicht wurde, kann es vorteilhaft sein, die aus den Membraneinheiten erhaltenen zwei oder mehr Retentatströme zu vereinigen und in einer weiteren Membraneinheit nochmals zu entwässern. Eine solche bevorzugte Ausführungsart des erfindungsgemäßen Verfahrens wird in Fig. 3 schematisch dargestellt. Auch diese Ausführungsart des erfindungsgemäßen Verfahrens wird bevorzugt so durchgeführt, dass vor Vereinigung der beiden Ströme die Wassergehalte durch Trennung in den individuellen Membraneinheiten auf nahezu den gleichen Wert (siehe oben) gebracht werden.

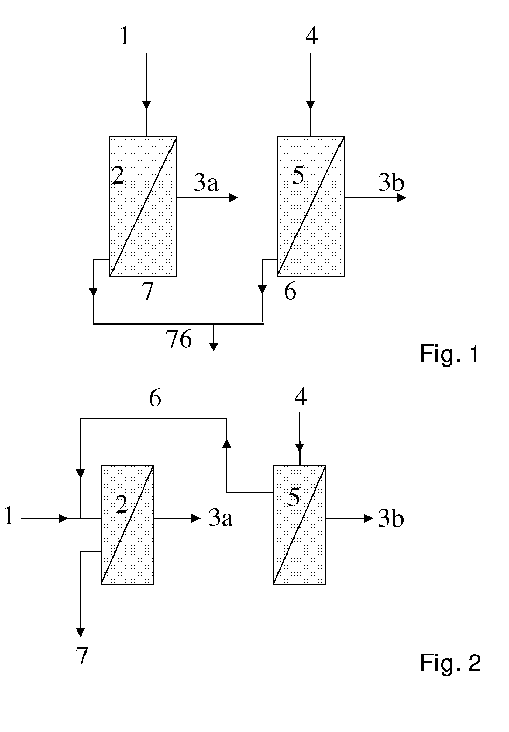

In einer anderen Ausführungsform des erfindungsgemäßen Verfahrens wird der Retentatstrom aus der Membraneinheit zur Trennung eines Ethanolstroms mit einem höheren Wassergehalt mit dem Zustrom einer Membraneinheit zur Trennung eines Ethanolstroms mit einem niedrigeren Wassergehalt vereinigt. Dies kann insbesondere dann vorteilhaft sein, wenn zumindest zwei wasserreichere Ethanolströme eingesetzt werden, die einen relativ großen Unterschied bezüglich der Wasserkonzentration, z. B. größer 2,5 Massen-% oder vorzugsweise größer 5 Massen-% und besonders bevorzugt von 5 bis 10 Massen-%, aufweisen, wobei der Wassergehalt des Ethanolstroms mit dem kleineren Gehalt an Wasser vorzugsweise eine Konzentration aufweist, die schon relativ nah an der Spezifikationskonzentration liegt. In diesem Fall wird aus dem Ethanolstrom mit dem höheren Wassergehalt in der Membraneinheit möglichst soviel Wasser entfernt, dass das erhaltene Retentat einen Wassergehalt aufweist, der sich maximal um 1 Massen-%, vorzugsweise um weniger als 0,5 Massen-% vom Wassergehalt des Ethanolstroms mit dem geringeren Wassergehalt unterscheidet und der Retentatstrom wird mit dem Ethanolstrom mit geringem Wassergehalt vereinigt und als Zustrom in die Membraneinheit zur Entwässerung des Ethanolstroms mit dem geringeren Wassergehalt geführt. Eine solche Ausführungsart des erfindungsgemäßen Verfahrens wird in Fig. 2 schematisch dargestellt.

Im einfachsten Fall einer Ausführungsform des erfindungsgemäßen Verfahrens werden die Retentate einfach vereinigt bevor sie einer weiteren Verwendung zugeführt werden. Eine solche Ausführungsform ist in Fig. 1 dargestellt.

Wie bereits erwähnt, kann das Verfahren der vorliegenden Erfindung ein integraler Teil einer ETBE-Anlage sein, wobei mit dem Verfahren z. B. der Einsatzalkohol und im ETBE-Prozess anfallendes Rückethanol entwässert werden können. Ein beispielhaftes ETBE-Verfahren, in welches das Verfahren der vorliegenden Erfindung integriert sein kann, wird weiter unten beschrieben.

In einer besonderen Ausführungsform des erfindungsgemäßen Verfahrens kann das erfindungsgemäß hergestellte an Wasser arme Ethanol als Edukt in einer ETBE-Synthese verwendet werden und das überschüssige Ethanol aus dem Prozess der ETBE-Synthese durch Extraktion mit Wasser aus dem ETBE-Prozess entfernt werden und dieser Extraktionsstrom direkt oder nach einer destillativen Aufarbeitung als wasserreicherer Ethanolstrom einer Membraneinheit zugeführt werden. Als zweiter wasserreicherer Ethanolstrom kann insbesondere der Einsatzalkohol dienen, der handelsüblich einen Wassergehalt im Bereich der Azeotropkonzentration aufweist.

Der Einsatzalkohol, der im erfindungsgemäßen Verfahren als wasserhaltiger Ethanolstrom eingesetzt werden kann, kann bevorzugt ein Ethanol/Wasser-Azeotrop-Gemisch mit einem Wassergehalt von 4,4 Massen-% oder ein Gemisch, das einen geringeren Wassergehalt aufweist, sein. Die Wassergehalte im Einsatzalkohol können bevorzugt im Bereich von 4,4 bis 0,5 Massen-%, insbesondere im Bereich von 1 bis 0,6 Massen-% liegen. Darüber hinaus kann das Frischethanol höhere Alkohole (Fuselöle) bis zu 1 Massen-% enthalten.

Der Rückethanol, der im erfindungsgemäßen Verfahren als wasserhaltiger Ethanolstrom eingesetzt werden kann, kann z. B. dadurch gewonnen werden, dass bei einem ETBE-Verfahren aus einem Ethanol enthaltenden $C_4$-Kohlenwasserstoffgemisch mit Wasser oder einer wässrigen Lösung Ethanol extrahiert wird. Aus dem wässrigen Extrakt kann durch Destillation das Ethanol abgetrennt werden. Das dabei als Rückethanol gewonnene Ethanol/Wasser-Gemisch entspricht bestenfalls dem Azeotrop mit einem Wassergehalt von zumindest 4,4 Massen-%. Meistens liegt der Wassergehalt aber höher. Üblicherweise beträgt der Wassergehalt des Rückethanols zwischen 4,4 und 12 Massen-%, insbesondere zwischen 6 und 10 Massen-%.

In einer bevorzugten Ausführungsform des erfindungsgemäßen Verfahrens kann das Rückethanol, das aus einem Ethanol-haltigen Kohlenwasserstoffgemisch durch Extraktion mit Wasser oder einer wässrigen Lösung entfernt und aus dem Extrakt durch Destillation z. B. als Ethanol/Wasser-Azeotrop oder als ein Gemisch aus Ethanol/Wasser-Azeotrop und zusätzlichem Wasser gewonnen wird, mit einem oder mehreren der in den Membraneinheiten anfallenden Permeate, die häufig Wassergehalte größer 40 Massen-% aufweisen, gemeinsam aufgearbeitet werden. Die Aufarbeitung kann wie oben beschrieben durch Trennung an Membranen, durch Destillation oder durch Destillation unter Verwendung eines Schleppmittels erfolgen. Optional können ein oder mehrere Permeate zusammen mit dem undestillierten ethanolhaltigen Wasserextrakt aus einer ETBE-Anlage aufgearbeitet werden.

Mit dem erfindungsgemäßen Verfahren kann ein Strom wasserärmeres Ethanol, insbesondere eine Zusammensetzung enthaltend Ethanol und geringe Mengen an Wasser erhalten werden, welche/s vorzugsweise einen Wassergehalt von kleiner 3 Massen-%, bevorzugt kleiner 1 Massen-%, besonders bevorzugt kleiner 0,5 Massen-% und ganz besonders bevorzugt kleiner 0,3 Massen-% aufweist. Bevorzugt weist die Zusammensetzung, hergestellt nach dem erfindungsgemäßen Verfahren einen Ethanol mit einem Wassergehalt von kleiner 1 Massen-%, besonders bevorzugt kleiner 0,3 Massen-% auf. Erfindungsgemäß hergestellte Zusammensetzungen von wasserarmem Ethanol, insbesondere Zusammensetzungen, welche einen Wassergehalt von kleiner 1 Massen-%, besonders bevorzugt kleiner 0,3 Massen-% aufweisen, können insbesondere als Edukt zur Herstellung von ETBE verwendet werden. Insbesondere werden solche Zusammensetzungen bzw. wird ein solches Ethanol bei Verfahren eingesetzt, bei denen die Umsetzung durch saure Ionenaustauscherharze katalysiert wird. Die ETBE-Herstellung kann in einer oder mehreren Reaktionsstufen erfolgen. Verfahren zur Herstellung von ETBE können z. B. EP 0 071 032, US 6,472,568, RU 2 168 490, RU 2 167 143, US 6,107,526 oder US 5,990,361 entnommen werden, auf die ausdrücklich verwiesen wird. Ein zweistufiges ETBE-Verfahren, in dem das erfindungsgemäß entwässerte Ethanol bevorzugt eingesetzt werden kann, wird nachfolgend beschrieben.

Das bevorzugte Verfahren zur Herstellung von ETBE aus technischen Mischungen von $C_4$-Kohlenwasserstoffen I, die mindestens 1-Buten, Isobuten, n-Butan und 2-Butene enthalten, zeichnet sich dadurch aus, dass es die Verfahrensschritte

a) Umsetzung von Teilen des in der technischen Mischung enthaltenen Isobutens mit Ethanol zu ETBE in Gegenwart eines sauren Katalysators,
b) Abtrennung der nicht umgesetzten $C_4$-Kohlenwasserstoffe III aus dem Austrag der Stufe a) durch thermische Trennverfahren unter Erhalt einer (im Wesentlichen) ETBE aufweisenden Fraktion II,
c) destillative Trennung der $C_4$-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) Umsetzung des in Fraktion IV enthaltenen Isobutens mit Ethanol VI in Gegenwart saurer Katalysatoren zu ETBE und
e) Abtrennung der nicht umgesetzten $C_4$-Kohlenwasserstoffe VIII aus dem Austrag von Stufe d) unter Erhalt einer ETBE aufweisenden Fraktion VII,

aufweist.

Verfahrensschritt a)

Vorzugsweise wird der Verfahrensschritt a) so durchgeführt, dass der Umsatz des Isobutens im Verfahrensschritt a) von größer 50 %, vorzugsweise größer 70 %, bevorzugt größer 80 %, besonders bevorzugt größer 90 % und ganz besonders bevorzugt größer 95 % beträgt. Die Höhe des Umsatzes an Isobuten kann z. B. durch die Anzahl der in Schritt a) verwendeten Reaktoren oder durch Wahl von geeigneten Reaktionsbedingungen, die der Fachmann leicht durch einfache Vorversuche ermitteln kann, gesteuert werden.

Die Veretherung des Isobutens wird als sauer katalysierte Reaktion durchgeführt. Als Ethanol kann hochreiner Ethanol, reiner Ethanol oder Ethanol verwendet werden, der geringe Mengen an Verunreinigungen aufweist. Bevorzugt liegt die Reinheit des eingesetzten Ethanols, angegeben in Massen-% an Ethanol, größer 90 %, besonders bevorzugt größer 95 % und ganz besonders bevorzugt gleich oder größer 99 %. Ethanol, welcher eine Reinheit von größer-gleich 99 Massen-% aufweist, kann z. B. Bioethanol sein. Der Gehalt an Wasser liegt bevorzugt unter 3 Massen-%, besonders bevorzugt unter 1 Massen-%, ganz besonders bevorzugt unter 0,5 Massen-%. Besonders bevorzugt wird als Ethanol vergällter Ethanol eingesetzt. Ganz besonders bevorzugt wird als Ethanol ein Ethanol eingesetzt, welches als Vergällungsmittel ETBE, vorzugsweise in einer Konzentration von 0 bis 5 Massen-%, bevorzugt von 0,005 bis 1 Massen-%, besonders bevorzugt 0,05 bis 1 Massen-% und ganz besonders bevorzugt von 0,01 bis 0,2 Massen-% aufweist. In Deutschland wird besonders bevorzugt Ethanol eingesetzt, welches mindestens von 0,1 bis 1 Massen-% Vergällungsmittel aufweist. Ganz besonders bevorzugt wird wasserarmes Ethanol oder eine wasserarmes Ethanol aufweisende Zusammensetzung eingesetzt, die durch das erfindungsgemäße Verfahren erhalten wurde.

Für die Umsetzung von Isobuten mit Alkoholen, insbesondere mit Methanol zu Methyl-tert.-butylether, wurden diverse Verfahrensvarianten entwickelt (vergleich: Ullmann's Encyclopedia of Industrial Chemistry, Online Version, 2004, Wiley & Sons, Stichwort Methyl Tert-Butyl Ether, und dort zitierte Literatur; Obenaus, Fritz; Droste, Wilhelm, Erdoel & Kohle, Erdgas, Petrochemie (1980), 33(6), 271 bis 275; DE 26 29 769; DE 28 53 769). Prinzipiell sind alle diese Verfahren zur Umsetzung des Isobutens mit Alkoholen als Verfahrensschritt a) geeignet. Bevorzugt werden Verfahren, bei denen die Umsetzung in flüssiger Phase an einem sauren Ionenaustauscherharz erfolgt.

Als Reaktoren, in denen das Ethanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren etc.) eingesetzt werden. Sie können mit oder ohne partieller Rückführung, wobei gegebenenfalls der Rückführungsstrom gekühlt werden kann, betrieben werden.

In einer bevorzugten Ausführungsform wird die Umsetzung des Isobutens mindestens zweistufig durchgeführt, wobei die erste Stufe als adiabatischer Festbettreaktor mit Rückführung (Schlaufenreaktor) und die folgenden Stufen als Festbettstufen ohne Rückführung und/oder als Reaktivdestillation betrieben werden. Das Verhältnis von rückgeführter Menge zu Frischzulauf ($C_4$-Kohlenwasserstoffe und Ethanol) liegt bevorzugt bei 0,5 bis 20 t/t, besonders bevorzugt bei 1 bis 5 t/t und ganz besonders bevorzugt bei 2 bis 3 t/t. Die Reaktoren können bei Temperaturen von vorzugsweise 10 bis 160 °C, bevorzugt von 30 bis 110°C, betrieben werden. Der Druck in den Festbettstufen beträgt vorzugsweise 5 bis 50 bar$_{absolut}$ (bara), bevorzugt 7,5 bis 20 bara und besonders bevorzugt von 8 bis 13 bara. Der Kreislaufreaktor weist vorzugsweise eine Eingangstemperatur von 35 bis 50 °C und eine Ausgangstemperatur von 50 bis 70 °C auf und wird bevorzugt bei 10 bis 13 bara betrieben. Da das thermodynamische Gleichgewicht zwischen Ethanol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben. Besonders bevorzugt wird im Verfahrensschritt a) ein Reaktorsystem eingesetzt, welches drei in Reihe geschaltete Reaktoren aufweist, von denen der erste Reaktor als Schlaufenreaktor betrieben wird und die beiden nachfolgenden Reaktoren im geraden Durchgang betrieben werden. Es kann vorteilhaft sein, wenn in dem Verfahrensschritt a) mehrere, vorzugsweise zwei dieser Reaktorsysteme vorhanden sind, so dass bei Reparaturarbeiten, wie z. B. Katalysatorwechsel, an einem Reaktor eines des Reaktorsysteme der Verfahrensschritt a) in dem anderen Reaktorsystem ohne Unterbrechung der Verfahrens (allerdings unter Halbierung des Durchsatzes) weiter durchgeführt werden kann. Bei Verwendung von Reaktorsystemen aus drei Reaktoren werden die Reaktoren vorzugsweise bei einer Temperatur von 30 bis 80 °C, bevorzugt 40 bis 75 °C und einem Druck von 5 bis 20 bara, bevorzugt 7 bis 15 bara betrieben, wobei vorzugsweise die Temperatur in den Reaktoren vom ersten zum letzten Reaktor abfällt. Die Reaktoren nach dem Kreislaufreaktor weisen vorzugsweise eine Eingangstemperatur von 30 bis 50 °C und eine Ausgangstemperatur von 35 bis 45 °C auf und werden bevorzugt ebenfalls bei 8 bis 13 bara betrieben.

Das molare Verhältnis von Ethanol zu Isobuten beträgt im Verfahrensschritt a) vorzugsweise von 5 zu 1 bis 0,9 zu 1, bevorzugt von 2 zu 1 bis 1 zu 1 und besonders bevorzugt von 1,2 zu 1 bis 1 zu 1. Da im Verfahrensschritt a) ein geringerer Umsatz an Isobuten akzeptiert werden kann, kann im Vergleich zu Verfahrensschritt d) ein geringerer Ethanolüberschuss vorteilhaft sein.

In einer bevorzugten Ausführungsform wird die Addition des Ethanols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt a) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus dem Isobutenhaltigen technischen Kohlenwasserstoffgemisch I und Ethanol ein Reaktionsgemisch hergestellt, das hinsichtlich seiner Isobuten-, Ethanol- und ETBE-Konzentration in der Nähe des thermodynamischen Gleichgewichts liegt. Der Umsatz des Isobutens beträgt dabei bevorzugt mehr als 90 %. Dieses Gemisch wird in der nächsten/letzten Reaktionsstufe in die Reaktivdestillationskolonne eingespeist, wo ein weiterer Teil des Isobutens zum Ether umgesetzt wird. Besonders bevorzugt erfolgt die Durchführung des Verfahrensschrittes a) in einem Reaktorsystem, welches drei in Reihe geschaltete Reaktoren, vorzugsweise Festbettreaktoren, von denen der erste vorzugsweise in Schlaufen-fahrweise und die beiden nachfolgenden Reaktoren im geraden Durchgang betrieben werden, und eine Reaktivdestillation aufweist, wobei der Reaktoraustrag des letzten der in Reihe geschalteten Reaktoren in die Reaktivdestillation geführt wird.

Die Umsetzung des Isobutens mit Ethanol zum ETBE erfolgt in der Reaktivdestillation in Abhängigkeit vom Druck vorzugsweise im Temperaturbereich von 40 bis 140 °C, bevorzugt von 60 bis 90 °C, besonders bevorzugt von 65 bis 85 °C (Temperatur im Bereich der Kolonne, in der sich der Katalysator befindet. Die Sumpftemperatur der Kolonne kann deutlich höher liegen). Die Reaktivdestillationskolonne wird vorzugsweise bei Drücken, gemessen am Kolonnenkopf, von 3 bara bis 15 bara, bevorzugt 7 bara bis 13 bara betrieben, insbesondere von 8 bara bis 11 bara.

Weist der Verfahrensschritt a) des bevorzugten ETBE-Verfahrens eine Reaktivdestillation auf, so wird, wie in DE 101 02082 für MTBE beschrieben, das Isobuten aufweisende $C_4$-Kohlenwasserstoffgemisch zusammen mit Ethanol in den ersten der Vorreaktoren eingespeist. Dabei wird der Ethanol bevorzugt im Überschuss eingesetzt. In den Vorreaktoren entsteht ein Gemisch, in dem Isobuten, Ethanol und ETBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Ethanol enthalten sein, als für die vollständige Umsetzung des noch vorhandenen Isobutens gebraucht wird. Der Ethanolüberschuss sollte jedoch derart bemessen werden, dass eine ausreichende Ethanolmenge für das sich bildende Azeotrop aus Ethanol und $C_4$-Kohlenwasserstoffen vorhanden ist.

Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Optional kann, wenn der Ethanolgehalt im Kolonnenzulauf zur Reaktivdestillationskolonne unter dem maximal zulässigen Wert liegt, zusätzlicher Ethanol zugemischt werden. Darüber hinaus kann am Kopf der Reaktivdestillationskolonne oberhalb des Kolonnenzulaufs unterhalb eines Flüssigkeitsverteilers oder in einem Flüssigkeitsverteiler oberhalb oder im Bereich der Reaktivzone, vorzugsweise im Bereich der Reaktivzone über eine gesonderte Einrichtung eine Ethanol-Einspeisung erfolgen. Eine zusätzliche Einspeisung von Ethanol kann z. B. in den Rücklauf der Kolonne oder direkt in die reaktiven Packungen erfolgen. Die zusätzliche Ethanolzugabe sollte so bemessen sein, dass in den Packungen der Reaktivzone der Ethanolgehalt in der Flüssigphase vorzugsweise größer-gleich 1,5 Massen-%, bevorzugt größer-gleich 2 Massen-% und besonders bevorzugt von 2 bis 3 Massen-% beträgt. Durch die Zugabe von Ethanol in die Reaktionszone wird sichergestellt, dass trotz der Abreaktion ausreichend Ethanol als Edukt zur Verfügung steht.

Bevorzugt weist die Reaktivdestillationskolonne oberhalb der Katalysatorpackung einen Bereich rein destillativer Trennung auf, besonders bevorzugt mit 5 bis 20, insbesondere mit 7 bis 10 theoretische Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators kann vorzugsweise von 12 bis 36, insbesondere von 20 bis 30 theoretische Trennstufen umfassen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 30 bis 98 %, vorzugsweise von 50 bis 95 % und besonders bevorzugt von 75 bis 90 % bezogen auf den Isobutengehalt im Zulauf zur Reaktivdestillation erreicht wird.

Die hydraulische Belastung in der katalytischen Packung der Kolonne beträgt vorzugsweise 10 % bis 110 %, bevorzugt 20 % bis 90 % und besonders bevorzugt 35 bis 75 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden (Vauck/Müller, "Grundoperationen chemischer Verfahrenstechnik", S. 626, VEB Deutscher Verlag für Grundstoffindustrie). Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen kleiner als 1,5 betrieben, insbesondere mit solchen, die größer 0,6 und kleiner 1,2, bevorzugt größer 0,7 und kleiner 1,1 betragen.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht. Es ist im bevorzugten ETBE-Verfahren aber auch möglich, diese Bereiche räumlich zu trennen, ohne auf die Vorteile einer Reaktivdestillation zu verzichten.

In einer Verfahrensvariante kann die Reaktivdestillationskolonne als Destillationskolonne mit einem oder mehreren außenliegenden Reaktoren, die den Katalysator enthalten und im Nebenstrom betrieben werden, sogenannten Seitenreaktoren, ausgeführt sein.

Das Kopfprodukt der Reaktivdestillationskolonne enthält im Wesentlichen ein $C_4$-Kohlenwasserstoffgemisch und Ethanol.

Als Katalysator wird vorzugsweise sowohl in den Festbettstufen als auch in einer gegebenenfalls vorhandenen Reaktivdestillationskolonne, ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen keine sauren Stoffe an das Produktgemisch abgeben, weil dies zu Ausbeuteverluste führen kann.

Die Aktivität der Katalysatoren wird vorzugsweise so gewählt, dass sie unter Reaktionsbedingungen die Addition von Ethanol an Isobuten katalysieren, jedoch kaum die Addition an lineare Butene. Weiterhin sollten die Katalysatoren die Oligomerisierung von linearen Butenen und die Dialkyletherbildung aus zwei Molekülen eingesetzten Ethanol möglichst nicht oder nur wenig katalysieren. Im Hinblick auf eine hohe Ausbeute an 1-Buten sollte die Aktivität für die Isomerisierung von 1-Buten zu 2-Buten vorzugsweise gering sein.

Als feste Katalysatoren können beispielsweise Zeolithe, säureaktivierte Bentonite und/oder Tonerden, sulfonierte Zirkoniumoxide, Montmorillonite oder saure Ionenaustauscherharze verwendet werden.

Eine im ETBE-Verfahren in Verfahrensschritt a) bevorzugte Gruppe von eingesetzten sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im ETBE-Verfahren können die Ionenaustauscherharze in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, vorzugsweise von 0,7 bis 2,0 eq/l, insbesondere von 1,1 bis 2,0 eq/l, bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

Im Reaktionsteil einer gegebenenfalls in Verfahrensschritt a) vorhandenen Reaktivdestillation können die gleichen Katalysatoren eingesetzt werden, wie sie in den einfachen Reaktoren eingesetzt werden. In der Reaktivdestillationskolonne kann der Katalysator entweder in der Packung integriert sein, beispielsweise KataMax® (wie in EP 0 428 265 beschrieben), KataPak® (wie in EP 0 396 650 oder DE 298 07 007.3 U1 beschrieben) oder auf Formkörpern aufpolymerisiert sein (wie in US 5 244 929 beschrieben).

Verfahrensschritt b)

Die Abtrennung der nicht umgesetzten $C_4$-Kohlenwasserstoffe III aus dem Austrag des Schrittes a) in Schritt b) erfolgt durch thermische Trennverfahren, beispielweise durch Destillation oder Fraktionierung. Beinhaltet der Verfahrensschritt a) eine Reaktivdestillation, so kann der Verfahrensschritt b) teilweise oder vollständig bereits bei der Durchführung der Reaktivdestillation stattfinden und ein separater Schritt b) kann gegebenenfalls entfallen.

Der Verfahrensschritt b) kann vorzugsweise in einer Destillationskolonne durchgeführt werden. Vorzugsweise weist die Destillationskolonne eine Anzahl an theoretischen Trennstufen von 25 bis 50, bevorzugt 30 bis 40 auf. Der Zulauf zu dieser Kolonne erfolgt vorzugsweise im Bereich der 10-ten bis 15-ten theoretischen Trennstufe (von oben). Die Destillation im Verfahrensschritt a) wird vorzugsweise bei einem Druck von 3 bis 10, bevorzugt von 4 bis 7 bara, einer bevorzugten Kopftemperatur von 30 bis 70 °C, besonders bevorzugt von 45 bis 60 °C und einer bevorzugten Sumpftemperatur von 105 bis 125 °C, besonders bevorzugt von 110 bis 120 °C durchgeführt.

Das thermische Trennverfahren wird vorzugsweise so durchgeführt, dass als Sumpfprodukt eine im Wesentlichen ETBE aufweisende Fraktion und als Kopfprodukt eine im Wesentlichen nicht umgesetzte $C_4$-Kohlenwasserstoffe sowie Ethanol enthaltende Fraktion erhalten wird. Werden die Butenoligomeren vor der Umsetzung mit Ethanol abgetrennt, besteht das Sumpfprodukt der Reaktivdestillationskolonne bevorzugt aus ETBE.

Das Kopfprodukt des Verfahrensschrittes b), also das Kopfprodukt der Reaktivdestillation oder der thermischen Trennung, kann direkt einer weiteren Auftrennung gemäß Verfahrensschritt c) zugeführt werden oder aber zunächst in einem oder mehreren Aufarbeitungsschritten aufgearbeitet werden.

Verfahrensschritt f)

Das bevorzugte ETBE-Verfahren weist vorzugsweise zwischen den Verfahrensschritten b) und c) einen weiteren Verfahrensschritt f) auf, in dem aus dem Kopfprodukt des Verfahrensschrittes b) also dem Kopfprodukt aus der Reaktivdestillation oder aus der thermischen Trennung, welches im Wesentlichen nicht umgesetzte $C_4$-Kohlenwasserstoffe und Ethanol aufweist, zunächst das Ethanol vollständig oder nahezu vollständig von den nicht umgesetzten $C_4$-Kohlenwasserstoffen abgetrennt wird.

Bevorzugt wird das Kopfprodukt aus dem Verfahrensschritt b) welches am Kopf der Destillationskolonne oder Reaktivdestillationskolonne erhalten wird, in eine Extraktionskolonne überführt, in die im Gegenstrom als Extraktionsmittel Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion der Strom aus in Stufe a) und gegebenenfalls b) nicht umgesetzten Kohlenwasserstoffen III erhalten. Dieses Produkt kann in Verfahrensschritt c) eingespeist werden.

Der Verfahrensschritt f) kann vorzugsweise in einer Extraktionskolonne durchgeführt werden. Vorzugsweise weist die Extraktionskolonne von 5 bis 20, bevorzugt von 10 bis 15 theoretische Trennstufen auf. Die Extraktion im Verfahrensschritt f) wird vorzugsweise bei einem Druck von 5 bis 12, bevorzugt von 7 bis 10 bara. Die Extraktion im Verfahrensschritt f) wird vorzugsweise bei einer Temperatur von 30 bis 60 °C und bevorzugt von 35 bis 45 °C durchgeführt. Dass Verhältnis vom Extraktionsmittel Wasser zum Kopfprodukt aus dem Verfahrensschritt b) bzw. a) beträgt vorzugsweise von 0,05 bis 0,5, bevorzugt von 0,1 bis 0,25 und besonders bevorzugt von 0,15 bis 0,2.

Das im Sumpf der Extraktionskolonne anfallende mit Ethanol angereicherte Extraktionsmittel kann destillativ aufgetrennt werden und das so erhaltene Ethanol als wasserreicher Ethanolstrom dem erfindungsgemäßen Verfahren zugeführt werden, in welchem es zu wasserärmerem Ethanol aufgearbeitet wird, welches dem ETBE-Verfahren als Ausgangsstoff in den Schritte a) oder d) wieder zugeführt werden.

Verfahrensschritt c)

Nach der Abtrennung des ETBE und gegebenenfalls nach Abtrennung des nicht umgesetzten Ethanols, wird der aus Schritt b) oder f) erhaltene Kohlenwasserstoffstrom in Schritt c) destillativ getrennt. Die destillative Trennung wird so durchgeführt, dass eine mindestens 1 Buten und Isobuten enthaltende Fraktion IV (Kopffraktion) und eine nahezu Isobuten-freie, vorzugsweise weniger als 5 Massen-%, bevorzugt weniger als 1 Massen-% und besonders bevorzugt weniger als 0,1 Massen-% Isobuten aufweisende, mindestens 2-Butene und n-Butan enthaltende Fraktion V (Sumpffraktion), erhalten wird. Die Fraktion V enthält mindestens 95 Massen-%, vorzugsweise mindestens 99 Massen-%, besonders bevorzugt mindestens 99,8 Massen-% der ursprünglich im als Produkt des Schrittes c) erhaltenen Kohlenwasserstoffstroms enthaltenen 2-Butene. Bevorzugt weist die Fraktion IV weniger als 1 Massen-%, besonders bevorzugt weniger als 0,2 Massen-% an n-Butan auf. Die destillative Trennung kann in für die Auftrennung solcher Kohlenwasserstoffgemische üblicherweise eingesetzten Apparaturen durchgeführt werden. Solche Apparaturen können z. B. Destillations- oder Fraktionierungskolonnen sein.

Bevorzugt wird die Trennung in einer Superfraktionierkolonne durchgeführt. Der Zulauf zu dieser Kolonne erfolgt vorzugsweise in der unteren Hälfte, bevorzugt im unteren Drittel der Kolonne. Wegen der engen Siedelage des zu trennenden Gemisches wird die Destillation vorzugsweise in einer Kolonne mit vorzugsweise mehr als 100, bevorzugt mehr als 125, besonders bevorzugt mit 150 oder mehr theoretischen Trennstufen und ganz besonders bevorzugt mit 150 bis 200 theoretischen Trennstufen durchgeführt. Das Rücklaufverhältnis (Rücklaufmenge zu Destillatabnahme) beträgt, in Abhängigkeit von der realisierten Stufenzahl und vom Betriebsdruck, vorzugsweise kleiner-gleich 20, bevorzugt kleiner-gleich 14, besonders bevorzugt kleiner-gleich 11 und ganz besondere bevorzugt von 8 bis 11. Die Kondensation kann gegen Kühlwasser oder Luft durchgeführt werden. Der Destillatbehälter wird vorzugsweise als flüssigflüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Die Trennung gemäß Verfahrensschritt c) wird vorzugsweise bei einem Druck von 4 bis 10 bara, bevorzugt bei einem Druck von 5 bis 7 bara durchgeführt. Die Temperatur bei welcher die Trennung durchgeführt wird beträgt vorzugsweise von 35 bis 65 °C, bevorzugt 40 bis 50 °C.

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten.

Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem ETBE-Verfahren, aber auch eine Kolonnen, die außerhalb des ETBE-Verfahrens am Anlagenstandort vorhanden ist, mit der Kolonne des Verfahrensschrittes c) verschaltet werden.

Verfahrensschritt d)

Die aus Schritt c) erhaltene Isobuten-haltige Fraktion IV wird im bevorzugten ETBE-Verfahren in einem weiteren Reaktionsschritt (Schritt d) umgesetzt, bei der das restliche Isobuten durch Anlagerung von Ethanol zum ETBE umgesetzt wird.

Die Veretherung des Isobutens wird ebenso wie die Veretherung gemäß Schritt a) als sauer katalysierte Reaktion durchgeführt. Als Ethanol kann hochreiner Ethanol, reiner Ethanol oder Ethanol verwendet werden, der geringe Mengen an Verunreinigungen aufweist. Bevorzugt liegt die Reinheit des eingesetzten Ethanols, angegeben in Massen-% an Ethanol, größer 90%, besonders bevorzugt größer 95 % und ganz besonders bevorzugt bei gleich oder größer 99 %. Ethanol mit einer Reinheit von größer-gleich 99 Massen-% wird in Europa beispielsweise als Bioethanol angeboten. Der Gehalt an Wasser liegt bevorzugt unter 3 Massen-%, besonders bevorzugt unter 1 Massen-%, ganz besonders bevorzugt unter 0,5 Massen-%. Es kann im Verfahren vorteilhaft sein, vergälltes Ethanol einzusetzen. Besonders bevorzugt wird als Ethanol ein Ethanol eingesetzt, welches als Vergällungsmittel ETBE, vorzugsweise in einer Konzentration von 0 bis 5 Massen-%, bevorzugt von 0,005 bis 1 Massen-%, besonders bevorzugt 0,05 bis 1 Massen-% und ganz besonders bevorzugt von 0,01 bis 0,2 Massen-% aufweist. In Deutschland wird vorzugsweise Ethanol eingesetzt, welches 0,1 bis 1 Massen-% an Vergällungsmittel aufweist. Durch die Verwendung von mit ETBE vergälltem Ethanol wird vermieden, dass Fremdstoffe in den Prozess eingetragen werden. Wiederum wird auch hier bevorzugt wasserarmes Ethanol oder eine Zusammensetzung, enthaltend wasserarmes Ethanol, eingesetzt, das bzw. die gemäß dem erfindungsgemäßen Verfahren erhalten wurde.

Für die Umsetzung von Isobuten mit Alkoholen, insbesondere mit Methanol zu Methyl-tert.-butylether wurden diverse Verfahrensvarianten entwickelt (vergleiche: Ullmann's Encyclopedia of Industrial Chemistry, Online Version, 2004, Wiley & Sons, Stichwort Methyl-tert-Butylether, und dort zitierte Literatur; Obenaus, Fritz; Droste, Wilhelm, Erdoel & Kohle, Erdgas, Petrochemie (1980), 33(6), 271 bis 275; DE 26 29 769; DE 28 53 769). Prinzipiell sind alle bekannten Verfahren zur Umsetzung des Isobutens mit Alkoholen geeignet als Verfahrensschritt d) eingesetzt zu werden.

Bevorzugt werden Verfahren, bei denen die Umsetzung in flüssiger Phase an einem sauren Ionenaustauscherharz erfolgt, eingesetzt. Als Reaktoren, in denen der Ethanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Sie können mit oder ohne partieller Rückführung, wobei gegebenenfalls der Rückführungsstrom gekühlt werden kann, betrieben werden. Besonders bevorzugt wird in Schritt d) ein Reaktorsystem eingesetzt, das zwei Reaktoren, insbesondere Festbettreaktoren aufweist. Vorzugsweise werden beide Reaktoren im geraden Durchgang betrieben.

Die Reaktoren können bei Temperaturen von 25 bis 110 °C, bevorzugt bei Temperaturen von 30 bis 70 °C und besonders bevorzugt bei Temperaturen von 35 bis 50 °C betrieben werden. Der Druck beträgt vorzugsweise 5 bis 50 bara, bevorzugt 10 bis 20 bara und besonders bevorzugt 10 bis 13 bara. Da das thermodynamische Gleichgewicht zwischen Ethanol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben.

Das molare Verhältnis von Ethanol zu Isobuten im Zulauf zum Verfahrensschritt d) liegt vorzugsweise im Bereich von 25 zu 1 bis 1 zu 1, besonders bevorzugt von 15 zu 1 bis 3 zu 1 und besonders bevorzugt im Bereich von 10 zu 1 bis 5 zu 1.

Als Katalysatoren können bevorzugt solche eingesetzt werden, wie sie für den Verfahrensschritt a) beschrieben werden, wobei das bevorzugte ETBE-Verfahren so durchgeführt werden kann, dass in Schritt a) und d) jeweils der gleiche Katalysator oder unterschiedliche Katalysatoren eingesetzt werden können. Vorzugsweise werden in den Schritten a) und d) die gleichen Katalysatoren eingesetzt.

In einer bevorzugten Ausführungsform wird die Addition des Ethanols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt d) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus der Isobuten-haltigen Fraktion IV und dem Ethanol VI ein Reaktionsgemisch hergestellt, das hinsichtlich seiner Isobuten-, Ethanol- und tert.-Butylether-Konzentration in der Nähe des thermodynamischen Gleichgewichts liegt. In diesem Reaktionsschritt wird der Restgehalt an Isobuten vorzugsweise so weit umgesetzt, dass mit der nachfolgenden Reaktivdestillation die erforderliche Reinheit des 1-Butens erreicht werden kann. Dieses Gemisch wird in der nächsten/letzten Reaktionsstufe in die Reaktivdestillationskolonne eingespeist, wo ein weiterer Teil des Isobutens zum Ether umgesetzt wird. Ganz besonders bevorzugt wird der Schritt d) in einem Reaktorsystem durchgeführt, das zwei in Reihe geschaltete Reaktoren und eine Reaktivdestillationskolonne aufweist, wobei die beiden Reaktoren vorzugsweise im geraden Durchgang betrieben werden, und der Austrag aus dem zweiten Reaktor in die Reaktivdestillationskolonne eingespeist wird.

Im Reaktionsteil der Reaktivdestillationskolonne können die gleichen Katalysatoren, wie die oben für die einfache Ausführungsform der Verfahrensstufe ohne die Verwendung einer Reaktivdestillation beschriebenen, eingesetzt werden.

In der Reaktivdestillationskolonne kann der Katalysator entweder in der Packung integriert sein, beispielsweise KataMax® (wie in EP 0 428 265 beschrieben), KataPak® (wie in EP 0 396 650 oder DE 298 07 007.3 U1 beschrieben) oder auf Formkörpern aufpolymerisiert (wie in US 5,244,929 beschrieben) sein.

Die Umsetzung des Isobutens mit Ethanol zu ETBE erfolgt in der Reaktivdestillation im Temperaturbereich von 10 bis 140 °C, bevorzugt bei 30 bis 70 °C, besonders bevorzugt bei 35 bis 50°C (Temperatur im Bereich der Kolonne, in der sich der Katalysator befindet. Die Sumpftemperatur der Kolonne kann deutlich höher liegen).

Insbesondere wird das ETBE durch Umsetzung mit Ethanol in einer Weise hergestellt, wie es in DE 101 02 082 für die Reaktion von Methanol mit Isobuten zu MTBE beschrieben wird. Das Isobuten aufweisende $C_4$-Kohlenwasserstoffgemisch wird zusammen mit Ethanol in den/die Vorreaktor(en) eingespeist. Dabei wird der Ethanol bevorzugt im Überschuss eingesetzt. In den Vorreaktoren entsteht ein Gemisch, in dem Isobuten, Ethanol und ETBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Ethanol enthalten sein, als für die vollständige Umsetzung des noch vorhandenen Isobutens gebraucht wird. Der Alkoholüberschuss sollte jedoch derart bemessen werden, dass eine ausreichende Ethanolmenge für das sich bildende Azeotrop aus Ethanol und $C_4$-Kohlenwasserstoffen vorhanden ist.

Optional kann, beispielsweise wenn der Ethanolgehalt im Kolonnenzulauf unter dem maximal zulässigen Wert liegt, zusätzlicher Ethanol zum Kolonnenzulauf zugemischt werden. Darüber hinaus kann am Kopf der Reaktivdestillationskolonne oberhalb des Kolonnenzulaufs unterhalb eines Flüssigkeitsverteilers oder in einem Flüssigkeitsverteiler oberhalb oder im Bereich der Reaktivzone, vorzugsweise im Bereich der Reaktivzone über eine gesonderte Einrichtung eine Ethanol-Einspeisung erfolgen. Eine zusätzliche Einspeisung von Ethanol kann z. B. in den Rücklauf der Kolonne oder direkt in die reaktiven Packungen erfolgen. Die zusätzliche Ethanolzugabe sollte so bemessen sein, dass in den Packungen der Reaktivzone der Ethanolgehalt in der Flüssigphase vorzugsweise größer-gleich 1,5 Massen-%, bevorzugt größer-gleich 2 Massen-% und besonders bevorzugt von 2 bis 3 Massen-% beträgt.

Bevorzugt weist die Reaktivdestillationskolonne oberhalb der Katalysatorpackung einen Bereich rein destillativer Trennung auf, besonders bevorzugt mit 5 bis 20, insbesondere mit 7 bis 10 theoretische Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators kann vorzugsweise von 12 bis 36, insbesondere von 20 bis 30 theoretische Trennstufen umfassen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Restisobutengehalt im Kopfprodukt von kleiner 2000 Massen-ppm (wppm), bevorzugt kleiner 1500 wppm erreicht wird.

Der Zulauf zur Reaktivdestillationskolonne kann oberhalb oder unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Die Reaktivdestillationskolonne wird bei Drücken, gemessen am Kolonnenkopf, von 3 bara bis 10 bara, bevorzugt 4 bara bis 7 bara betrieben, insbesondere von 5 bara bis 6 bara. Die hydraulische Belastung in der katalytischen Packung der Kolonne beträgt vorzugsweise 10 % bis 110 %, bevorzugt 20 % bis 90 % und besonders bevorzugt 35 bis 75 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden ( Vauck/Müller, "Grundoperationen chemischer Verfahrenstechnik", S. 626, VEB Deutscher Verlag für Grundstoffindustrie.).

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen von 0,2 bis 4 betrieben, insbesondere mit solchen, die von 0,4 bis 2, bevorzugt von 0,5 bis 1 betragen.

Wird in der Stufe d) als letzter Schritt eine Reaktivdestillationskolonne eingesetzt, so kann in dieser zumindest teilweise der Schritt d) sowie der Schritt e), nämlich die Abtrennung des ETBE von den nicht umgesetzten Kohlenwasserstoffen stattfinden. Auf einen weiteren Schritt e) kann dann gegebenenfalls verzichtet werden.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht. Es ist im bevorzugten ETBE-Verfahren aber auch möglich, diese Bereiche räumlich zu trennen, ohne auf die Vorteile einer Reaktivdestillation zu verzichten.

In einer Verfahrensvariante wird die Reaktivdestillationskolonne als Destillationskolonne mit einem oder mehreren außenliegenden Reaktor(en), der/die den Katalysator enthält/enthalten und im Nebenstrom betrieben wird/werden, ausgeführt.

Verfahrensschritt e)

Wird in Verfahrensschritt d) keine Reaktivdestillationskolonne zur Veretherung und gleichzeitigen Trennung eingesetzt, so muss im bevorzugten ETBE-Verfahren ein eigenständiger Schritt e) vorgesehen werden, in welchem das Produkt aus dem Verfahrensschritt d) getrennt wird in den ETBE enthaltenden Sumpfstrom und einen Strom, der die nicht umgesetzten Kohlenwasserstoffe aufweist. Ansonsten erfolgt die destillative Trennung gemäß Verfahrensschritt e) in der Reaktivdestillationskolonne. Die Trennung kann z. B. dadurch erfolgen, dass der Austrag aus dem Reaktor des Verfahrensschrittes d) in eine Destillationskolonne eingespeist wird. Die Kolonne kann mit einem Sumpfverdampfer und einem Kondensator für das Kopfprodukt ausgerüstet sein. Als Sumpfprodukt der Destillationskolonne wird ETBE und gegebenenfalls überschüssiger Ethanol erhalten. Das Kopfprodukt kann teilweise als Rücklauf in die Kolonne zurückgefahren werden. Der andere Teil, kann dem Verfahrensschritt h) zugeführt werden.

Die Kolonne weist vorzugsweise mehr als 20, bevorzugt mehr als 25, besonders bevorzugt von 30 bis 50 theoretischen Trennstufen auf. Das Rücklaufverhältnis ist, in Abhängigkeit von der realisierten Stufenzahl, vorzugsweise kleiner-gleich 1. Besonders bevorzugt wird das Rücklaufverhältnis auf einen Wert von 0,9 bis 0,6 eingestellt. Die Kondensation kann gegen Kühlwasser oder Luft durchgeführt werden. Zur Beheizung des Verdampfers der Kolonne kann z. B. Dampf eingesetzt werden. Es kann vorteilhaft sein, den Zulaufstrom zur Kolonne zumindest teilweise vorverdampft in die Kolonne zu leiten oder direkt in die Kolonne zu flashen. Bevorzugt wird dafür dem Zulaufstrom in einem externen Wärmeübertrager Wärme, z. B. durch Nutzung von Abwärme, zugeführt. Zum Erzielen einer teilweisen Verdampfung ist ein Kettle-Verdampfer die bevorzugte Ausführungsform des Wärmeübertragers. Es kann außerdem vorteilhaft sein, wenn ein mit Prozess- oder Abwärme auf niedrigerem Temperaturniveau beheizter Zwischenverdampfer im unteren Teil der Kolonne eingesetzt wird.

Der Zulauf zu der Kolonne erfolgt in Verfahrensschritt e) vorzugsweise auf der 10 bis 15 theoretischen Trennstufe. Die Kolonne wird vorzugsweise mit einem Druck von 4 bis 11, bevorzugt von 5 bis 8 bara betrieben. Die Kopftemperatur der in Verfahrensschritt e) eingesetzten Kolonne beträgt vorzugsweise von 40 bis 70 °C, bevorzugt von 45 bis 60 °C.

Weist das Kopfprodukt von Verfahrensschritt e) unabhängig davon, ob der Schritt in einer Destillations- oder Reaktivdestillationskolonne durchgeführt wurde, noch Restmengen an Ethanol in den $C_4$-Kohlenwasserstoffen auf, so kann es vorteilhaft sein, diese in mindestens einem zusätzlichen Extraktionsschritt mit Wasser auszuwaschen. Diese Wäsche kann nach den bekannten Standardverfahren der Technik, beispielsweise in einer Extraktionskolonne oder in einer Kaskade von Mixern und Trennbehältern, durchgeführt werden (Siehe Verfahrensschritt h)).

Das Sumpfprodukt, welches ETBE und gegebenenfalls überschüssiges Ethanol enthält, kann direkt verwendet werden oder einer Aufarbeitung, z. B. einer weiteren Destillation zugeführt werden, in welcher das ETBE von den übrigen Bestandteilen abgetrennt wird.

In einer weiteren bevorzugten Ausführungsform des Verfahrens wird das in der Reaktivdestillation bzw. Destillation der Schritte d) bzw. e) anfallende Sumpfprodukt, welches ETBE und gegebenenfalls nicht umgesetzten Ethanol enthält, ganz oder teilweise in den Schritt a) und/oder b) zurückgeführt. Diese Ausführungsform ist insbesondere dann vorteilhaft, wenn die Umsetzung in Schritt d) mit einem stöchiometrischen Überschuss an Ethanol betrieben wird. Bevorzugt wird bei dieser Variante in Schritt d) die Umsetzung nur in Festbettreaktoren durchgeführt und in Schritt e) eine destillative Trennung in einer Destillationskolonne durchgeführt. Die nicht umgesetzten $C_4$-Kohlenwasserstoffe VIII werden als Kopfprodukt erhalten und das anfallende Sumpfprodukt, welches mindestens ETBE und gegebenenfalls nicht umgesetzten Ethanol enthält, wird ganz oder teilweise in den Schritt a) und/oder b) zurückgeführt. Wird der Verfahrensschritt d) mit einem großen Ethanolüberschuss durchgeführt, z. B. mit einem molaren Verhältnis von Ethanol zu Isobuten von größer 2 zu 1, insbesondere größer 5 zu 1, so fällt als Sumpfprodukt in Schritt e) eine Mischung an, die im Wesentlichen Ethanol enthält und deshalb besonders gut als Einsatzstoff in die Stufe a) zurückgeführt werden kann. Bei dieser Ausführungsform wird das ETBE ausschließlich als Sumpfprodukt des Verfahrenschrittes b) erhalten. Zur destillativen Aufarbeitung des Sumpfproduktes aus Verfahrenschritt e) wird bei dieser Ausführungsform der Verfahrensschritt b) genutzt.

Verfahrensschritt h)

Es kann vorteilhaft sein, wenn das Kopfprodukt aus dem Verfahrensschritt d) bzw. e), welches am Kopf der Destillationskolonne oder Reaktivdestillationskolonne erhalten wird, in eine Extraktionskolonne überführt wird, in die im Gegenstrom als Extraktionsmittel Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion der Strom aus in Stufe d) und gegebenenfalls e) nicht umgesetzten Kohlenwasserstoffen VIII erhalten. Dieser kann einer weiteren Verwendung beispielsweise einer Aufarbeitung zu 1-Buten (Verfahrensschritt i)) zugeführt werden.
Der Verfahrensschritt h) kann vorzugsweise in einer Extraktionskolonne durchgeführt werden. Vorzugsweise weist die Extraktionskolonne von 5 bis 20, bevorzugt 10 bis 15 theoretische Trennstufen auf. Die Extraktion im Verfahrensschritt h) wird vorzugsweise bei einem Druck von 5 bis 12, bevorzugt von 7 bis 10 bara durchgeführt. Die Extraktion im Verfahrensschritt h) wird vorzugsweise bei einer Temperatur von 30 bis 60 °C, besonders bevorzugt von 35 bis 45 °C durchgeführt. Dass Verhältnis von Extraktionsmittel, insbesondere Wasser zum Kopfprodukt aus dem Verfahrensschritt d) bzw. e) beträgt vorzugsweise von 0,05 bis 0,5, bevorzugt von 0,1 bis 0,25 und besonders bevorzugt von 0,15 bis 0,2.
Das im Sumpf der Extraktionskolonne anfallende mit Ethanol angereicherte Extraktionsmittel kann destillativ aufgetrennt werden und das so erhaltene Ethanol als wasserreicher Ethanolstrom dem erfindungsgemäßen Verfahren zugeführt werden, in welchem es zu wasserärmerem Ethanol getrocknet wird, welches dem ETBE-Verfahren als Ausgangsstoff in den Schritte a) oder d) wieder zugeführt werden.

Verfahrensschritt i)

Aus dem aus der Reaktivdestillation bzw. Destillation gemäß Schritt e) erhaltenen und ggf. von Ethanol befreiten $C_4$-Kohlenwasserstoffgemisch VIII aus nicht umgesetzten Kohlenwasserstoffen, das im Wesentlichen 1-Buten, Isobutan und Leichtsieder aufweist, kann destillativ 1-Buten als weiteres Wertprodukt abgetrennt werden. Die Abtrennung des 1-Butens erfolgt vorzugsweise durch Destillation des Gemisches VIII in einer oder mehreren Destillationskolonnen.
In einer bevorzugten Ausführungsform erfolgt die Abtrennung des 1-Butens in einer Destillationskolonne, in der als Sumpfprodukt sehr reines 1-Buten erhalten wird. Als Kopfprodukt wird eine Isobutan-reiche Fraktion erhalten, die zudem Leichtsieder (beispielsweise $C_3$-Kohlenwasserstoffe) enthalten kann.
Bevorzugt wird die Trennung in einer Superfraktionierkolonne durchgeführt. Der Zulauf zu dieser Kolonne erfolgt vorzugsweise in die obere Hälfte, bevorzugt in die untere Hälfte der oberen Hälfte der Kolonne. Wegen der engen Siedelage des zu trennenden Gemisches wird die Kolonne mit vorzugsweise mehr als 100, bevorzugt mehr als 125, besonders bevorzugt mit 150 oder mehr und ganz besonders bevorzugt mit 150 bis 200 theoretischen Trennstufen durchgeführt. Das Rücklaufverhältnis (Rücklaufmenge zu Destillatabnahme) beträgt, in Abhängigkeit von der realisierten Stufenzahl und vom Betriebsdruck, vorzugsweise kleiner-gleich 100, bevorzugt kleiner 70, besonders bevorzugt von 30 bis 60. Die Kondensation kann gegen Kühlwasser oder Luft durchgeführt werden. Der Destillatbehälter wird vorzugsweise als Flüssigkeit-Flüssigkeit-Abscheider ausgeführt. Dadurch kann gegebenenfalls im Zulaufstrom enthaltenes Wasser als zweite Phase im Destillatbehälter abgetrennt und es kann ein technisch wasserfreies Sumpfprodukt erhalten werden.
Die Trennung gemäß Verfahrensschritt i) wird vorzugsweise bei einem Druck von 5 bis 11 bara, bevorzugt bei einem Druck von 6 bis 8 bara durchgeführt. Die Kopftemperatur, bei welcher die Trennung durchgeführt wird, beträgt vorzugsweise von 35 bis 65 °C, bevorzugt 45 bis 50 °C. Wenn eine Wärmeintegration vorgesehen wird, kann es vorteilhaft sein, dass der Verfahrensschritt i) bei höherer Temperatur und damit höherem Druck durchgeführt wird.
Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem ETBE-Verfahren, aber auch eine Kolonne, die außerhalb des ETBE-Verfahrens am Anlagenstandort vorhanden ist, mit der Kolonne des Verfahrensschrittes i) verschaltet werden. Besonders bevorzugt ist die zweite Kolonne die $C_4$-Trennkolonne aus Verfahrensschritt c). Dabei wird eine der Kolonnen bei so hohem Druck gefahren, dass ihre Kondensationstemperatur zur Beheizung der anderen Kolonne ausreicht.

In einer weiteren bevorzugten Ausführungsform werden in einer ersten Destillationskolonne Leichtsieder als Kopfprodukt abgetrennt, im Sumpf der Kolonne wird eine Mischung, die hauptsächlich 1-Buten und Isobutan enthält, erhalten. In einer zweiten Kolonne wird diese Sumpfmischung in 1-Buten, welches als Sumpfprodukt anfällt, und eine Isobutan-reiche Fraktion (Kopfprodukt), getrennt.

Mit dem bevorzugten ETBE-Verfahren hergestelltes, reines 1-Buten enthält vorzugsweise weniger als 5000 wppm (Massen-ppm), bevorzugt weniger als 2000 wppm und besonders bevorzugt weniger als 1500 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd. Eine weitere Verwendung des hergestellten nahezu Isobuten-freien 1-Butens ist die Herstellung von n-ButenOligomeren, insbesondere nach dem Octol-Prozess.

In Verfahrensschritt i) fällt neben dem 1-Buten üblicherweise (je nach Ausgangszusammensetzung der $C_4$-Kohlenwasserstoffe) eine Isobutan-reiche Fraktion an. Diese kann weiter, vorzugsweise zu reinem Isobutan, aufgereinigt werden. Eine Aufreinigung zu reinem Isobutan kann beispielsweise durch vollständige Hydrierung der noch enthaltenen Alkene zu Alkanen und anschließende Destillation erfolgen. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine.

Einsatzstoffe ETBE-Verfahren

In dem bevorzugten ETBE-Verfahren können alle üblicherweise zur Verfügung stehenden technischen $C_4$-Kohlenwasserstoffgemische eingesetzt werden. Geeignete Isobuten-haltige $C_4$-Ströme sind beispielsweise $C_4$-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben. (K.Weissermel, H.J. Arpe, Industrielle Organische Chemie, Wiley-VCH, 5. Auflage, 1998, Seite 23-24; 65-99; 122-124).

Bevorzugt eingesetzt werden $C_4$-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, oder Katcrackern. Die als Nebenprodukt anfallenden $C_4$-Schnitte enthalten je nach CrackVerfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der $C_4$-Fraktion liegen bei $C_4$-Fraktionen aus Steam-Crackern bei 18 bis 35 Massen-%, bei -Fluid-Katcrackern (FCC) bei 10 bis 20 Massen-%.

Für das ETBE-Verfahren kann es vorteilhaft sein, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H. J. Arpe, Industrielle Organische Chemie, Wiley-VCH, 5. Auflage, 1998, Seiten 119 bis 121).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. in EP 0 523 482 beschrieben. Auch durch selektive Umsetzungen des 1,3-Butadiens, zum Beispiel Dimerisierung zum Cyclooctadien, Trimerisierung zum Cyclododecatrien, Polymerisations- oder Telomerisationsreaktionen, kann das 1,3-Butadien zumindest teilweise entfernt werden. Wurde ein Crack-$C_4$-Schnitt als Rohstoff eingesetzt, bleibt in allen Fällen ein Kohlenwasserstoffgemisch (Raffinat I oder hydriertes Crack-$C_4$ ($HCC_4$)) zurück, das hauptsächlich die gesättigten Kohlenwasserstoffe, n-Butan und Isobutan und die Olefine Isobuten, 1-Buten und 2-Butene enthält.

Bevorzugt wird in dem ETBE-Verfahren in einer zusätzlichen Reinigungsstufe, die einer oder mehreren der Verfahrensschritte a), b), c), d), e) oder f) vorgeschaltet wird, in den $C_4$-Kohlenwasserstoffströmen enthaltene mehrfach ungesättigten Kohlenwasserstoffe katalytisch selektiv hydriert. Besonders bevorzugt ist zumindest vor dem Verfahrensschritt a) oder c) und ganz besonders bevorzugt vor der Verfahrensstufe c) eine solche Reinigungsstufe vorgesehen, insbesondere wenn nicht ausgeschlossen werden kann, dass die eingesetzten technischen $C_4$-Kohlenwasserstoffströme mehrfach ungesättigte Kohlenwasserstoffe aufweisen.

Bei den mehrfach ungesättigten Kohlenwasserstoffen handelt es sich hauptsächlich um 1,3-Butadien; 1,2-Butadien, Butenin und 1-Butin sind, wenn überhaupt, in deutlich geringerer Menge enthalten. Die Hydrierung kann in einem ein- oder mehrstufigen Hydrierprozess in der Flüssigphase an einem Palladiumkontakt erfolgen. Zur Absenkung des Gehalts an 1,3-Butadien unter vorzugsweise 1000 wppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0,05 bis 100 Massen-ppm (wppm) zugesetzt wird. Der Gehalt an mehrfach ungesättigten Kohlenwasserstoffen sollte im Zulauf zu dieser Stufe unter 1 %, bevorzugt unter 0,5 %, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986,39,73).

Sind in den Isobuten-haltigen $C_4$-Strömen Mengen mehr als 1 % an mehrfach ungesättigten Kohlenwasserstoffe wie 1,3-Butadien enthalten, werden sie vorzugsweise in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-$C_4$ aus einem Steam-Cracker mit einem Gehalt an 1,3-Butadien von typischerweise 38 bis 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer Selektivhydrierung (SHP) erfolgen kann.

Die in dem ETBE-Verfahren eingesetzten Kohlenwasserstoffmischungen mit Isobuten und linearen Butenen weisen bevorzugt die folgenden Zusammensetzungen auf, wobei je nach Gehalt an ungesättigten Kohlenwasserstoffen eine Hydrierung oder Selektivhydrierung vor einem der Schritte a) bis d), vorzugsweise vor Schritt a) oder c) durchgeführt wird.

| Tabelle 1: Typische Zusammensetzungen von technischen Kohlenwasserstoffgemischen, die im ETBE-Verfahren eingesetzt werden können. | | | | | |
|---|---|---|---|---|---|
| | Steamcracker | | Steamcracker | | Katcracker |
| Komponente | $HCC_4$ | $HCC_4$ / SHP | Raff. I | Raff. I / SHP | $CC_4$ | $CC_4$ / SHP |
| Isobutan [Massen-%] | 1 - 4,5 | 1 - 4,5 | 1,5-8 8 | 1,5-8 8 | 36-37 | 36-37 |
| n-Butan [Massen-%] | 5-8 | 5-8 | 6-15 | 6-15 | 12-14 | 12-14 |
| trans-Buten [Massen-%] | 18-21 | 18-21 | 7-10 | 7-10 | 11-13 | 11-13 |
| 1-Buten [Massen-%] | 35-45 | 35-45 | 15-35 | 15-35 | 11-13 | 11-13 |
| Isobuten [Massen-%] | 22-28 | 22-28 | 33-50 | 33-50 | 14-16 | 14-16 |
| cis-Buten [Massen-%] | 5-9 | 5-9 | 4-8 | 4-8 | 10-12 | 10-12 |
| 1,3-Butadien [wppm] | 500 - 8000 | 0 - 50 | 50 - 8000 | 0 - 50 | < 10000 | 0 - 50 |

Erläuterung

- $HCC_4$: typisch für eine $C_4$ Mischung, die aus dem Crack-$C_4$ eines Steamcrackers (High Severity) nach der Hydrierung des 1,3-Butadiens ohne zusätzliche Moderation des Katalysators erhalten wird.

- $HCC_4$ / SHP: Zusammensetzung $HCC_4$, bei dem Reste an 1,3-Butadien in einer SHP weiter reduziert wurden.

- Raff. I (Raffinat I): typisch für eine $C_4$ Mischung, die aus dem Crack-$C_4$ eines Steamcrackers (High Severity) nach der Abtrennung des 1,3-Butadiens, beispielsweise durch eine NMP-Extraktivrektifikation, erhalten wird.

- Raff. I / SHP: Zusammensetzung Raff. I, bei dem Reste an 1,3-Butadien in einer SHP weiter reduziert wurden.

- $CC_4$: typische Zusammensetzung eines Crack-$C_4$, das aus einem Katcracker erhalten wird.

- $CC_4$ / SHP: Zusammensetzung $CC_4$, bei dem Reste an 1,3-Butadien in einer SHP weiter reduziert wurden.

Das Raffinat I bzw. $HCC_4$ ist, neben anderen, ein bevorzugt eingesetztes Isobuten-haltiges Kohlenwasserstoffgemisch im ETBE-Verfahren. Da Anlagen zur Aufarbeitung von $C_4$-Kohlenwasserstoffen in der Regel als Strang (Verbund mehrerer Anlagen) aufgebaut sind, ist es jedoch möglich, dass das Raffinat I bzw. $HCC_4$ vor dem Eintritt in das ETBE-Verfahren eine oder mehrere andere Prozessstufe(n) durchläuft. Diese Prozessstufe(n) kann/können beispielsweise auch ein Verfahren bzw. Verfahrensschritt(e) sein, wie sie in den Ausführungsformen zum Verfahrensschritt a) beschrieben worden sind. In dem ETBE-Verfahren einsetzbare $C_4$-Kohlenwasserstoffgemische können auch solche sein, wie sie aus Verfahren gemäß den Ausführungsformen der Verfahrensschritt a) und anschließender Trennung gemäß Verfahrensschritt b) erhalten werden. Insbesondere können auch solche Gemische eingesetzt werden, wie sie bei der Herstellung von tert.-Butanol (TBA) aus Isobuten nach Abtrennung des TBA erhalten werden. Auf diese Weise kann jeweils ein individuell angepasstes Gesamtkonzept zur Aufarbeitung mit dem entsprechenden Produktportfolio realisiert werden.

Typische Verfahren, die den ETBE-Verfahren vorgelagert sein können, sind Wasserwäschen, Reinigungsverfahren in Adsorbern, Trocknungsverfahren und Destillationen.

Wasserwäsche (ETBE-Verfahren)

Durch eine Wasserwäsche können hydrophile Komponenten aus dem einzusetzenden technischen Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestillation stammen können). Auch Sauerstoffverbindungen (z. B. Aceton aus FCC) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

Adsorber (ETBE-Verfahren)

Adsorber werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt.

Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb®).

Trocknung (ETBE-Verfahren)

Im Isobuten-haltigen Kohlenwasserstoffgemisch gegebenenfalls enthaltenes Wasser, das beispielsweise aus der Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen $C_4$-Kohlenwasserstoffen ausgenutzt werden oder es können Schleppmittel zugesetzt werden.

Die Trocknung des Kohlenwasserstoffgemisches kann aus diversen Gründen vorteilhaft sein, beispielsweise zur Verringerung der Bildung von Alkoholen (hauptsächlich tert.-Butylalkohol) in Schritt a) oder zur Vermeidung von technischen Problemen durch Abscheidung von Wasser oder zur Vermeidung von Eisbildung bei niedrigen Temperaturen (z. B. bei der Zwischenlagerung).

Destillation (ETBE-Verfahren)

Destillationsschritte können beispielsweise genutzt werden, um Verunreinigungen abzutrennen (beispielsweise Leichtsieder wie $C_3$-Kohlenwasserstoffe, Schwersieder wie $C_5$-Kohlenwasserstoffe) oder um Fraktionen mit unterschiedlichen Isobutenkonzentrationen zu erhalten. Dies kann sowohl direkt mit dem Raffinat I bzw. dem $HCC_4$ erfolgen oder nachdem eine oder mehrere andere Prozessstufe(n) durchlaufen wurde(n). Durch direkte Destillation des Raffinats I bzw. des $HCC_4$ ist beispielsweise eine Trennung in eine an 2-Butenen und n-Butan verarmte, Isobuten reichere Fraktion möglich.

Je nach Zusammensetzung des einzusetzenden technischen Kohlenwasserstoffgemisches und/oder nach den Reinheiten der Zielprodukte kann das technische Kohlenwasserstoffgemisch also direkt in den Schritt a) des bevorzugten ETBE-Verfahrens oder aber erst nach einer Vorbehandlung durch eines oder mehrere der vorgenannten Verfahren eingesetzt werden.

Mit dem bevorzugten ETBE-Verfahren lässt sich auf einfache Weise ETBE bzw. eine Zusammensetzung, die ETBE enthält, herstellen, welche/s als Sumpfprodukt der Reaktivdestillationen oder der Destillationen gemäß den Schritten b) und e), bevorzugt als Sumpfprodukt des Schrittes b) erhalten werden/wird.

Gegenstand der vorliegenden Erfindung ist deshalb insbesondere auch ein Verfahren zur Herstellung von ETBE, insbesondere das gerade beschriebene Verfahren zur Herstellung von ETBE, welches dadurch gekennzeichnet ist, dass ein gemäß dem erfindungsgemäßen Verfahren zur Herstellung von wasserärmeren Ethanol hergestelltes wasserärmeres Ethanol als Edukt in dem ETBE-Verfahren verwendet wird und dass überschüssiger Ethanol aus dem ETBE-Verfahren sowie Frischethanol als wasserreiche Ethanolströme mit unterschiedlichem Wassergehalt dem erfindungsgemäßen Verfahren zur Herstellung von wasserärmerem Ethanol zugeführt wird.

An Hand der Figuren Fig. 1 bis Fig. 3 wird das erfindungsgemäße Verfahren durch Blockschaltbildern näher erläutert, ohne dass das Verfahren auf die dort beispielhaft abgebildeten Ausführungsarten beschränkt sein soll. In den schematischen Darstellungen sind nur die wesentlichen Stufen dargestellt. Auf die Darstellung von verfahrenstechnisch üblichen Strömen, wie z. B. Kühlwasserströmen, Kreislaufströmen, oder Rückspeisungen, und/oder üblichen Apparaturen, wurde teilweise zu Gunsten einer besseren Übersicht verzichtet. Gewöhnliche Bauteile, wie beispielsweise Pumpen, Vakuumpumpen, Ventile, Wärmetauscher, die in den Blockschaltbildern nicht dargestellt sind, können jedoch Bauteile in einer Anlage zur Durchführung des erfindungsgemäßen Verfahrens sein.

Ein Blockschema einer Verfahrensvariante, mit der die Entwässerung der Ethanolströme nach dem erfindungsgemäßen Verfahren durchgeführt werden kann, ist in Fig. 1 dargestellt. Das Ethanol (4) mit dem höheren Wassergehalt wird in die Membraneinheit (5) eingeleitet. Dort wird ein Retentat (6), das den spezifizierten Wassergehalt aufweist, und ein wasserreiches Permeat (3b) erhalten. Der wasserärmere Ethanolstrom (1) wird in die Membraneinheit (2) eingeleitet. Als Retentat (7) wird Ethanol mit dem gewünschten Wassergehalt sowie ein Permeat (3a) erhalten. Die Retentate (6) und (7) werden zu einem gemeinsamen Strom (76) vereinigt, der vorzugsweise in einer ETBE-Anlage, z. B. als Edukt in den Veretherungs-reaktoren eingesetzt werden kann. Die Permeate (3a) und (3b) können getrennt oder bevorzugt gemeinsam aufgearbeitet werden. Eine Möglichkeit besteht in der destillativen Trennung in Wasser und in ein Kopfprodukt, das entweder aus dem Ethanol/Wasser-Azeotrop oder aus dem Ethanol/Wasser-Azeotrop und zusätzlichem Wasser besteht. Das Destillat kann an geeigneter Stelle in die Entwässerungsanlage rückgeführt werden, beispielsweise als Strom (4) oder als eine Teilmenge von Strom (4).

Ein Blockschema einer zweiten Verfahrensvariante, mit der die Entwässerung der Ethanolströme nach dem erfindungsgemäßen Verfahren durchgeführt werden kann, ist in Fig. 2 dargestellt. Das Ethanol (4) mit dem höheren Wassergehalt wird in die Membraneinheit (5) eingeleitet. Dort entsteht ein Retentat (6), das nahezu den gleichen Wassergehalt aufweist wie der Ethanolstrom (1), und ein wasserreiches Permeat (3b). Der wasserärmere Ethanolstrom (1) wird zusammen mit Retentat (6) in die Membraneinheit (2) eingeleitet. Als Retentat (7) wird Ethanol mit dem gewünschten Wassergehalt und ein Permeat (3a) erhalten. Das Retentat (7) wird vorzugsweise in einer ETBE-Synthese als Edukt eingesetzt. Die Permeate (3a) und (3b) können getrennt oder bevorzugt gemeinsam aufgearbeitet werden. Eine Möglichkeit besteht in der destillativen Trennung in Wasser und in ein Kopfprodukt, das entweder aus dem Ethanol/Wasser-Azeotrop oder aus dem Ethanol/Wasser-Azeotrop und zusätzlichem Wasser besteht. Das Destillat kann an geeigneter Stelle in die Entwässerungsanlage rückgeführt werden, beispielsweise als Strom (4) oder als eine Teilmenge davon.

Ein Blockschema einer weiteren Verfahrensvariante zeigt Fig. 3. In der Membraneinheit (2) wird der Ethanolstrom (1) in ein wasserärmeres Retentat (7) und in ein wasserreiches Permeat (3a) getrennt. In der Membraneinheit (5) wird der Ethanolstrom (4) in ein Retentat (6), das nahezu den gleichen Wassergehalt wie Retentat (7) aufweist, und ein wasserreiches Permeat (3b) getrennt. Die Retentate (7) und (6) werden zusammen in Membraneinheit (9) in einen Ethanolstrom (10) mit dem gewünschten Wassergehalt und in ein wasserreiches Permeat (3c) aufgetrennt. Der Ethanolstrom (10) wird vorzugsweise in einer ETBE-Anlage eingesetzt. Die Permeate (3a), (3b) und (3c) können getrennt oder bevorzugt gemeinsam aufgearbeitet werden. Eine Möglichkeit besteht in der destillativen Trennung in Wasser und in ein Kopfprodukt, das entweder aus dem Ethanol/Wasser-Azeotrop oder aus dem Ethanol/Wasser-Azeotrop und zusätzlichem Wasser besteht. Das Destillat kann an geeigneter Stelle in die Entwässerungsanlage rückgeführt werden, beispielsweise als Strom (4) oder eine Teilmenge davon.

Die nachfolgenden Beispiele sollen die Erfindung erläutern, ohne den Schutzbereich der sich aus den Patentansprüchen und der Beschreibung ergibt, einzuschränken.

Beispiele

Zur Illustration der Vorteile der erfindungsgemäßen Verschaltungen werden zwei Beispiele berechnet. Es sind zwei Ströme zu trocknen, wie sie in technischen Prozessen, insbesondere bei der ETBE-Herstellung anfallen können:

Ein Frisch-Ethanol-Strom von 10 t/h mit einem Wassergehalt von 1 Massen-%
Ein Rück-Ethanol-Strom von 1,45 t/h mit einem Wassergehalt von 9 Massen-%.
Ziel ist eine Rest-Konzentration von 0,3 Massen-% Wasser im getrockneten Ethanol.

Die Betriebstemperatur betrug 90 °C. Es wurde eine zur Ethanol-Trocknung gebräuchliche Polyvinylalkohol (PVA) Membrane eingesetzt. Für diesen Membran-Typ sind in der Literatur unfangreiche Messdaten publiziert (S. Klatt: Zum Einsatz der Pervaporation im Umfeld der chemischen Industrie, Shaker Verlag 1993; M. Franke: Auslegung und Optimierung von Pervaporationsanlagen zur Entwässerung von Lösungsmitteln und Lösungsmittelgemischen, Dissertation, Aachen, 1990). Bei 90 °C erreichen diese Membranen einen Reinwasserfluss von etwa 14 $kg/m^2h$ (Klatt, 1993). Kommerzielle PVA-Membranen sind unter Handelsnamen wie z. B. PERVAP 2200 (Sulzer Chemtech) frei erhältlich.

Nach Brüschke (Brüschke, H. E. A.: State of Art of Pervaporation Processes, In: Nunes, S. P. and Peinemann, K.-V. (Eds.): Membrane Technology in the chemical Industry, Wiley-VCH, 2001) erfolgte die Ermittlung der Membranfläche nach der Gleichung:

$$A = \overset{\bullet}{m}\,\frac{1}{J_0}\,\ln\left(\frac{C_{Feed}}{C_{Produkt}}\right)$$

Darin war A die gesuchte Membranfläche in m$^2$, m der Zulaufmassenstrom in kg/h, $J_0$ der extrapolierte Reinwasserfluss in kg/(m$^2$h) und $C_{Feed}$ sowie $C_{Produkt}$ waren die Konzentration des Zulaufs und des Retentats in Massen-%. Die Selektivität der üblichen Entwässerungs-Membranen ist über weite Konzentrationsbereiche konstant und musste für eine vergleichende Untersuchung, bei der in allen Varianten ähnliche Konzentrationsbereiche durchfahren werden, nicht berücksichtigt werden. Selektivitäten für die Entwässerungs-Membran PERVAP 2200 werden z. B. von Brüschke (2001) angegeben. Die Permeat-Aufarbeitung (Kondensation und VakuumAnlage) wurde in den berechneten Beispielen nicht weiter betrachtet.

Als Beispiel 1 (Vergleich) wird ein nicht erfindungsgemäßes Verfahren berechnet, in dem die beiden Zulaufströme (1) und (4) vor der Membran-Anlage gemischt werden. Damit ergibt sich eine mittlere Zulaufkonzentration von 2,01 Massen-% und eine nach der oben genannten Formel berechnete erforderliche Membranfläche von 1557 m$^2$.

Als Beispiel 2 wird ein erfindungsgemäßes Verfahren nach Fig. 2 berechnet, in dem das Rück-Ethanol (4) in einer eigenen Membran-Anlage (5) bis auf einen Wassergehalt von 1 Massen-% getrocknet wird. Dazu sind etwa 352 m$^2$ Membranfläche erforderlich. Anschließend wird das Retentat (6) mit dem Frisch-Ethanol (1) gemischt und einer zweiten Membran-Anlage (2) zugeführt, in der der Gesamtstrom die Wasser-Spezifikation von 0,3 Massen-% erreicht. Für die zweite Membran-Anlage ergibt sich dann eine Membranfläche von etwa 860 m$^2$. In Summe hat die Anlage nach Beispiel 2 eine Fläche von 1212 m$^2$. Die Permeat-Aufarbeitung erfolgt für die beiden Membran-Anlagen (2) und (5) gemeinsam.

Die Ergebnisse der Beispiele 1 und 2 sind in Tabelle 2 zusammengefasst. Der Vergleich der beiden Beispiele erlaubt unmittelbar die Beurteilung der Vorteile des erfindungsgemäßen Verfahrens. Ein großer Teil der Betriebskosten einer Membran-Anlage wird durch den regelmäßigen Membran-Ersatz nach abgelaufener Standzeit verursacht. Verfahrensbedingt sind bei Membran-Anlagen auch die anderen Betriebskosten wie Heizleistung, Betriebsmittel für die Vakuumanlage und die Permeat-Aufarbeitung in erster Näherung proportional zur Membranfläche. Mit insgesamt etwa 1212 m$^2$ ist die Membranfläche beim erfindungsgemäßen Verfahren ca. 22 % geringer als bei der in Beispiel 1 beschriebenen Vergleichsanlage. Damit ergeben sich folgende Vorteile (siehe auch Tabelle 2):

In Beispiel 2 liegen die Investitionskosten ca. 18 % unter denen der Anlage nach Beispiel 1.
In Beispiel 2 liegen die Betriebskosten ca. 22 % unter denen der Anlage nach Beispiel 1.

Tabelle 2: Ergebnisse der Rechnungen zu Beispiel 1 und Beispiel 2

| | Strom | Zulauf menge [kg/h] | Wasser-Gehalt Zulauf [Ma-%] | Wasser-Gehalt Produkt [Ma-%] | Membranfläche [m$^2$] | Membranfläche relativ [%] | Investkosten [%] | Betriebskosten [%] |
|---|---|---|---|---|---|---|---|---|
| Beispiel 1 (Vergleich) | Ströme (1) und (4) | 11450 | 2,0 | 0,3 | 1557 | 100,0 | 100,0 | 100,0 |
| Beispiel 2 (Fig. 1) | Strom (1) | 10000 | 1,0 | 0,3 | 860 | | | |
| | Strom (4) | 1450 | 9,0 | 0,3 | 352 | | | |
| | Summe | 11450 | | | 1212 | 77,9 | 82,0 | 78,0 |

**Patentansprüche**

1. Verfahren zur Herstellung eines wasserärmeren Ethanols aus mindestens zwei Strömen von wasserreicherem Ethanol, die einen unterschiedlichen Wassergehalt aufweisen, durch Entwässerung an Membranen,
   **dadurch gekennzeichnet,**
   **dass** die Ethanolströme mit unterschiedlichen Wassergehalten getrennt in verschiedene Membraneinheiten eingeleitet werden, wobei die Ethanolströme in den Membraneinheiten jeweils in wasserärmeres Ethanol als Retentat und wasserreicheres Permeat getrennt werden und der Retentatstrom aus zumindest einer Membraneinheit zur Trennung eines Ethanolstroms mit einem höheren Wassergehalt mit dem Zustrom und/oder dem Retentatstrom zumindest einer anderen Membraneinheit zur Trennung eines Ethanolstroms vereinigt wird, wobei nur Ströme vereinigt werden, die einen Unterschied in der Ethanolkonzentration von maximal 1 Massen-% absolut aufweisen.

2. Verfahren nach Anspruch 1,
   **dadurch gekennzeichnet,**
   **dass** die Entwässerung an den Membranen bei einer Temperatur von 20 bis 150 °C durchgeführt wird.

3. Verfahren nach Anspruch 1 oder 2,
   **dadurch gekennzeichnet,**
   **dass** die Entwässerung an den Membranen mit einem Differenzdruck von 0,001 bis 2 MPa durchgeführt wird.

4. Verfahren nach zumindest einem der Ansprüche 1 bis 3,
   **dadurch gekennzeichnet,**
   **dass** die Wasserabtrennung an den Membranen durch Umkehrosmose, Pervaporation oder Dampfpermeation erfolgt.

5. Verfahren nach zumindest einem der Ansprüche 1 bis 4,
   **dadurch gekennzeichnet,**
   **dass** der Differenzdruck durch Erzeugen eines Vakuums auf der Permeatseite der Membrane erzeugt wird.

6. Verfahren nach Anspruch 5,
   **dadurch gekennzeichnet,**
   **dass** der Differenzdruck durch Erzeugen eines Unterdrucks auf der Permeatseite aller eingesetzten Membrane durch eine gemeinsame Unterdruckanlage erzeugt wird.

7. Verfahren nach zumindest einem der Ansprüche 1 bis 6,
   **dadurch gekennzeichnet,**
   **dass** zumindest ein Strom an wasserärmerem Ethanol erhalten wird, der einen Wassergehalt von unter 1 Massen-% aufweist.

8. Verfahren nach zumindest einem der Ansprüche 1 bis 7,
   **dadurch gekennzeichnet,**
   **dass** zwei wasserreichere Ethanolströme mit unterschiedlichen Wassergehalten, deren Wasserkonzentrationen sich um mehr als 1,0 Massen-% unterscheiden, eingesetzt werden.

9. Verfahren nach zumindest einem der Ansprüche 1 bis 8,
   **dadurch gekennzeichnet,**
   **dass** zumindest ein wasserreicherer Ethanolstrom Frischalkohol ist und zumindest ein wasserreicherer Ethanolstrom aus einer ETBE-Anlage stammen.

10. Verfahren nach zumindest einem der Ansprüche 1 bis 9,
    **dadurch gekennzeichnet,**
    **dass** die bei der Entwässerung in den Membraneinheiten anfallenden wasserreichen Permeate getrennt aufgearbeitet werden.

11. Verfahren nach einem der Ansprüche 1 bis 9,
    **dadurch gekennzeichnet,**
    **dass** die bei der Entwässerung in den Membraneinheiten anfallenden wasserreichen Permeate zusammen aufgearbeitet werden.

**12.** Verfahren nach Anspruch 10 oder 11,
**dadurch gekennzeichnet,**
**dass** die Permeate destillativ in Wasser und zumindest ein Destillat, bestehend aus einem Ethanol/Wasser-Gemisch, getrennt werden und das Destillat als wasserreicherer Ethanolstrom in eine Membraneinheit zurückgeführt wird.

**13.** Verfahren nach zumindest einem der Ansprüche 10 bis 12,
**dadurch gekennzeichnet,**
**dass** die Permeate zusammen mit einem Ethanol-haltigen wässrigen Extrakt aus einer ETBE-Anlage destillativ aufgearbeitet werden und dass das an Ethanol reiche Destillat als wasserreicherer Ethanolstrom in eine Membraneinheit zurückgeführt wird.

**14.** Verfahren nach zumindest einem der Ansprüche 1 bis 13,
**dadurch gekennzeichnet,**
**dass** die Retentate, wenn sich deren Wasserkonzentrationen um weniger als 0,2 Massen-% unterscheiden, vereinigt werden.

**15.** Verfahren nach zumindest einem der Ansprüche 1 bis 14,
**dadurch gekennzeichnet,**
**dass** zwei oder mehr Retentatströme vereinigt werden und in einer weiteren Membraneinheit entwässert werden.

**16.** Verfahren nach einem der Ansprüche 1 und 2,
**dadurch gekennzeichnet,**
**dass** der Retentatstrom aus der Membraneinheit zur Trennung eines Ethanolstroms mit einem höheren Wassergehalt mit dem Zustrom einer Membraneinheit zur Trennung eines Ethanolstroms mit einem niedrigeren Wassergehalt vereinigt wird.

**17.** Verfahren nach zumindest einem der Ansprüche 1 bis 16,
**dadurch gekennzeichnet,**
**dass** das erfindungsgemäß hergestellte wasserärmere Ethanol als Edukt in einer ETBE-Synthese verwendet wird und das überschüssiges Ethanol aus dem Prozess der ETBE-Synthese durch Extraktion mit Wasser aus dem ETBE-Prozess entfernt wird und dieser Extraktionsstrom direkt oder nach einer destillativen Aufarbeitung als wasserreicher Ethanolstrom einer Membraneinheit zugeführt wird.

**18.** Verfahren zur Herstellung von ETBE,
**dadurch gekennzeichnet,**
**dass** ein gemäß einem Verfahren nach einem der Ansprüche 1 bis 17 hergestelltes wasserärmeres Ethanol als Edukt in dem ETBE-Verfahren verwendet wird und das überschüssiges Ethanol aus dem ETBE-Verfahren sowie Frischethanol als wasserreichere Ethanolströme dem Verfahren gemäß einem der Ansprüche 1 bis 17 zugeführt wird.

**19.** Zusammensetzung enthaltend Ethanol und Wasser, wobei der Gehalt an Wasser kleiner 1 Massen-% beträgt, hergestellt nach einem Verfahren gemäß einem der Ansprüche 1 bis 17.

**20.** Verwendung einer Ethanol aufweisenden Zusammensetzung, hergestellt gemäß einem der Ansprüche 1 bis 17, als Edukt zur Herstellung von ETBE.

**21.** Verwendung gemäß Anspruch 20,
**dadurch gekennzeichnet,**
**dass** eine Zusammensetzung gemäß Anspruch 19 eingesetzt wird.

Fig. 1

Fig. 2

Fig. 3

EP 1 826 193 A1

**Europäisches Patentamt**

**EUROPÄISCHER RECHERCHENBERICHT**

Nummer der Anmeldung

EP 06 12 4922

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | BALLWEG A H ET AL: "pervaporation membranes- an economical method to replace conventional dehydration and rectification columns in ethanol distilleries" PROCEEDINGS FIFTH INTERNATIONAL ALCOHOL FUEL TECHNOLOGY SYMPOSIUM, Bd. I, 1982, Seiten 97-106, XP009086757 Beispiele I-IV; Fig. 2-6 | 1-16 | INV. C07C29/74 C07C31/08 C07C43/04 C07C41/06 |
| Y | Beispiele I-IV; Fig. 2-6 | 17 | |
| X,D | EP 0 071 032 A1 (EUTECO IMPIANTI SPA [IT]; PETROFLEX IND & COM SA [BR]) 9. Februar 1983 (1983-02-09) Anspruch 1, Beispiel 5 | 18-21 | |
| Y,D | US 6 107 526 A (FREY STANLEY J [US] ET AL) 22. August 2000 (2000-08-22) Anspruch 1, 7, 9; Figur | 17 | |
| A | US 5 607 557 A (STREICHER CHRISTIAN [FR]) 4. März 1997 (1997-03-04) Anspruch 1-17; Fig. 1,2 | 1-21 | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| München | 23. Juli 2007 | GRAMMENOUDI, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 06 12 4922

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-07-2007

| Im Recherchenbericht angeführtes Patentdokument | Datum der Veröffentlichung | Mitglied(er) der Patentfamilie | | Datum der Veröffentlichung |
|---|---|---|---|---|
| EP 0071032 A1 | 09-02-1983 | AR | 228191 A1 | 31-01-1983 |
| | | BR | 8204380 A | 18-01-1983 |
| | | CA | 1224810 A1 | 28-07-1987 |
| | | DE | 3263381 D1 | 05-06-1985 |
| | | ES | 8305672 A1 | 16-07-1983 |
| | | IN | 156276 A1 | 08-06-1985 |
| | | IT | 1167481 B | 13-05-1987 |
| US 6107526 A | 22-08-2000 | KEINE | | |
| US 5607557 A | 04-03-1997 | DE | 69505583 D1 | 03-12-1998 |
| | | DE | 69505583 T2 | 18-03-1999 |
| | | EP | 0675099 A1 | 04-10-1995 |
| | | ES | 2126225 T3 | 16-03-1999 |
| | | FR | 2717798 A1 | 29-09-1995 |
| | | JP | 3787664 B2 | 21-06-2006 |
| | | JP | 7278035 A | 24-10-1995 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0071032 A
- US 6472568 B
- RU 2168490
- RU 2167143
- US 6107526 A
- US 5990361 A
- DE 2629769
- DE 2853769
- DE 10102082
- EP 0428265 A
- EP 0396650 A
- DE 29807007 U1
- US 5244929 A
- EP 0523482 A
- EP 0081041 A

**In der Beschreibung aufgeführte Nicht-Patentliteratur**

- Ullmann's Encyclopedia of Technical Chemistry. vol. 9, 634-635
- Industrial application of vapour permeation. **U. SANDER ; H. JANSSEN.** Journal of Membrane Science. Elsevier Science Publishers B. V, 1991, vol. 61, 113-129
- **A. H. BALLWEG ; H. E. A. BRÜSCHKE ; W. H. SCHNEIDER ; G. F. TUSEL.** Pervaporation Membranes. *Proceedings of Fifth International Alcohol Fuel Technology,* 1982, 97-106
- **H. E. A. BRÜSCHKE.** State of Art of Pervaporation. *Proceeding of Third International Conference on Pervaporation,* 1988, 2-11
- Ullmann's Encyclopedia of Industrial Chemistry. Wiley & Sons, 2004
- **OBENAUS ; FRITZ ; DROSTE ; WILHELM ; ERDOEL ; KOHLE ; ERDGAS.** *Petrochemie,* 1980, vol. 33 (6), 271-275
- **VAUCK/MÜLLER.** Grundoperationen chemischer Verfahrenstechnik. VEB Deutscher Verlag für Grundstoffindustrie, 626
- **J. MACKOWIAK.** Fluiddynamik von Kolonnen mit modernen Füllkörpern und Packungen für Gas/Flüssigkeitssysteme. Otto Salle Verlag, 1991
- **K.WEISSERMEL ; H.J. ARPE.** Industrielle Organische Chemie. Wiley-VCH, 1998, 23-2465-99122-124
- **K.WEISSERMEL ; H. J. ARPE.** Industrielle Organische Chemie. Wiley-VCH, 1998, 119-121
- **ERDÖL ; KOHLE ; ERDGAS.** *Petrochem.,* 1986, vol. 39, 73
- **S. KLATT.** Zum Einsatz der Pervaporation im Umfeld der chemischen Industrie. Shaker Verlag, 1993
- **M. FRANKE.** Auslegung und Optimierung von Pervaporationsanlagen zur Entwässerung von Lösungsmitteln und Lösungsmittelgemischen. Dissertation, 1990
- State of Art of Pervaporation Processes. **BRÜSCHKE, H. E. A.** Membrane Technology in the chemical Industry. Wiley-VCH, 2001